(19) Europäisches Patentamt
European Patent Office
Office européen des brevets

(11) Numéro de publication : **0 114 011**
**B1**

(12) # FASCICULE DE BREVET EUROPÉEN

(45) Date de publication du fascicule du brevet : **12.11.86**

(51) Int. Cl.[4] : **C 07 J 41/00**, C 07 B 59/00, G 01 N 33/60, G 01 N 33/74

(21) Numéro de dépôt : **83402389.7**

(22) Date de dépôt : **12.12.83**

(54) **Dérivés estratriéniques radioactifs marqués à l'iode, procédé et intermédiaires de préparation, application aux dosages radio-immunologiques et à la préparation d'antigènes, antigènes obtenus.**

(30) Priorité : **13.12.82 FR 8220845**

(43) Date de publication de la demande : **25.07.84 Bulletin 84/30**

(45) Mention de la délivrance du brevet : **12.11.86 Bulletin 86/46**

(84) Etats contractants désignés : **BE CH DE GB IT LI LU NL**

(56) Documents cités :
**FR-A- 2 250 745**
**FR-A- 2 397 426**
**GB-A- 1 604 864**
**US-A- 4 339 390**

(73) Titulaire : **ROUSSEL-UCLAF**
**35, boulevard des Invalides**
**F-75007 Paris (FR)**

(72) Inventeur : **Jouquey, Alain**
**137 rue des Pyrénées**
**F-75020 Paris (FR)**
Inventeur : **Touyer, Gaetan**
**54 rue Petit**
**F-75019 Paris (FR)**
Inventeur : **Salmon, Jean**
**8, rue du Général Castelnau**
**F-75015 Paris (FR)**
Inventeur : **Mouren, Michel**
**184 Avenue de Choisy**
**F-75013 Paris (FR)**

(74) Mandataire : **Markovic, Borivoj Département des Brevets ROUSSEL UCLAF et al**
**B.P. no 9**
**F-93230 Romainville (FR)**

**EP 0 114 011 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux dérivés estratriéniques radioactifs marqués à l'iode, leur procédé et leurs intermédiaires de préparation, leur application aux dosages immunologiques et à la préparation d'antigènes et les antigènes obtenus.

L'invention a ainsi pour objet des dérivés estratriéniques marqués à l'iode, sous forme d'isomères syn ou sous forme d'isomères anti, ou sous forme de mélange d'isomères syn et anti, de formule générale (I) :

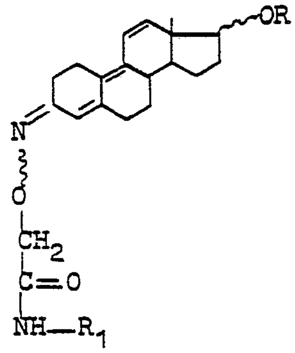

(I)

dans laquelle les lignes ondulées signifient que la fonction OR peut être en position α ou β et que la fonction oxime peut être en position syn ou anti, formule dans laquelle R représente un atome d'hydrogène, un groupement alcoyle ayant de 1 à 6 atomes de carbone ou un groupement acyle d'un acide carboxylique ayant de 2 à 12 atomes de carbone et $R_1$ représente le reste d'un acide aminé $R_1NH_2$ ou le reste d'un dérivé de ce dernier, ce reste étant accepteur d'iode et étant marqué à l'iode 125 ou 131.

Par reste d'un dérivé d'acide aminé, on entend de préférence le reste d'un dérivé décarboxylé d'un acide aminé ou le reste d'un ester d'alcoyle inférieur d'un acide aminé.

L'invention a plus particulièrement pour objet les dérivés estratriéniques radioactifs marqués à l'iode de formule générale (I) dans laquelle $R_1$ représente le reste d'un acide aminé $R_1NH_2$ ou le reste d'un dérivé de ce dernier, choisi dans le groupe constitué par l'histidine, la tyrosine, l'histamine, la tyramine et le tyrosinate de méthyle marqués à l'iode 125 ou 131.

Parmi les produits de formule générale (I), on citera notamment :

— la 17β-hydroxy-4,9,11-trièn-3-carboxyméthyloxime sous forme d'isomère syn, couplée à l'($^{125}$I) histamine de formule :

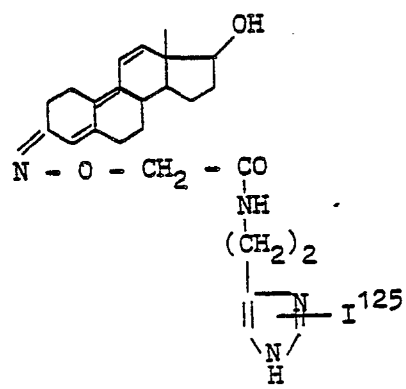

dans laquelle l'iode peut être en position 2 ou 5 ;

— la 17β-hydroxy-4,9,11-trièn-3-carboxyméthyloxime sous forme d'isomère anti, couplée à l'($^{125}$I) histamine de formule :

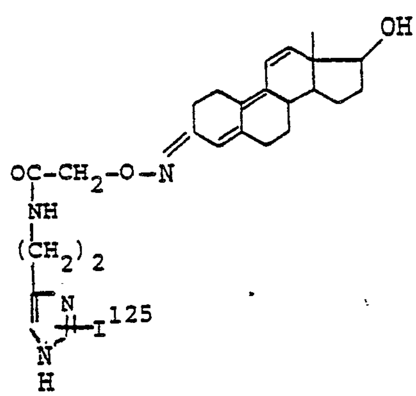

dans laquelle l'iode peut être en position 2 ou 5 ;

— la 17α-hydroxy-4,9,11-trièn-3-carboxyméthyloxime sous forme d'isomère syn, couplée à l'($^{125}$I) histamine de formule :

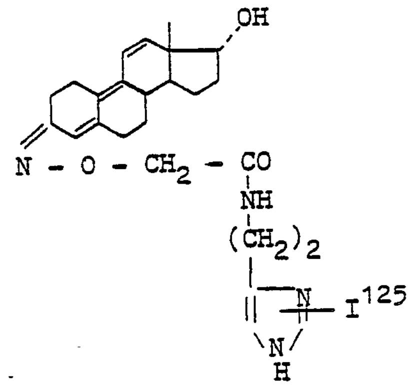

dans laquelle l'iode peut être en position 2 ou 5 ;

— la 17α-hydroxy-4,9,11-trièn-3-carboxyméthyloxime sous forme d'isomère anti, couplée à l'($^{125}$I) histamine de formule :

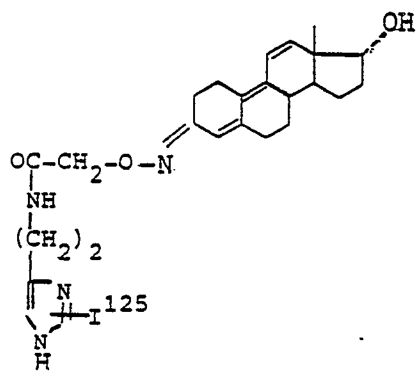

dans laquelle l'iode peut être en position 2 ou 5 ;

— la 17β- ou la 17α-hydroxy-4,9,11-trièn-3-carboxyméthyloxime sous forme de mélange d'isomère syn et anti couplée à l'($^{125}$I) histamine, l'iode pouvant être en position 2 ou 5.

L'invention a également pour objet un procédé de préparation des produits répondant à la formule générale ci-dessus.

Ce procédé est caractérisé en ce que :

— on fait réagir un produit de formule générale (II) :

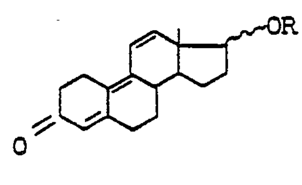

dans laquelle et dans ce qui suit R a les significations précitées, avec un halogénure de carboxyméthoxylamine en présence d'une base et obtient un produit de formule générale ($III_A$) :

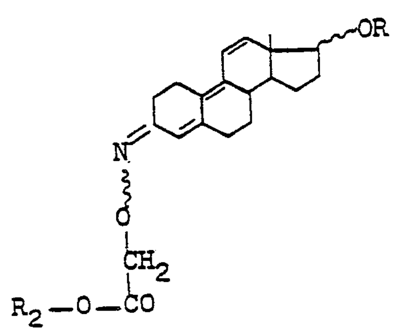

($III_A$)

dans laquelle la ligne ondulée sur l'azote indique que ce produit est sous forme de mélange d'isomères syn et anti et $R_2$ représente un atome d'hydrogène, puis :

— soit fixe sur la fonction acide de ce produit un groupement activateur de la fonction carbonyle et obtient le produit de formule générale ($III_C$) :

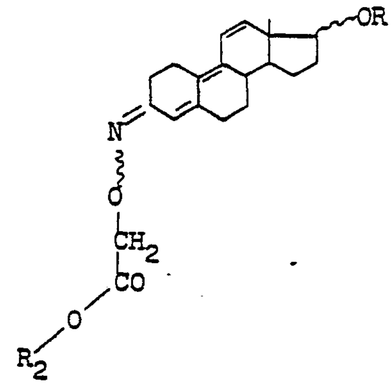

($III_C$)

dans laquelle $R_2$ représente un groupement activateur de la fonction carbonyle et la ligne ondulée sur l'azote indique que ce produit est sous forme de mélange d'isomères syn et anti, que l'on fait réagir avec un acide aminé accepteur d'iode marqué à l'iode 125 ou 131 ou avec un dérivé d'un tel acide et obtient le produit cherché de formule générale (I), que l'on isole sous forme de mélange d'isomères syn et anti, que l'on sépare éventuellement en ces isomères ;

— soit soumet le produit de formule ($III_A$), dans laquelle $R_2$ représente un atome d'hydrogène et la ligne ondulée sur l'azote indique que ce produit est sous forme de mélange d'isomères syn et anti, à l'action d'un agent d'estérification, pour obtenir un produit de formule générale ($III_B$) :

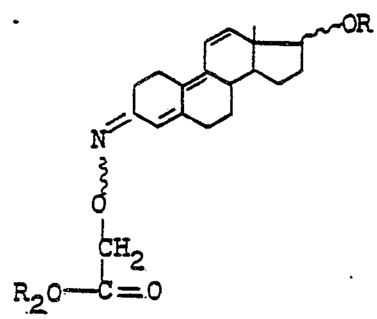

($III_B$)

dans laquelle $R_2$ représente un groupement alcoyle ayant de 1 à 6 atomes de carbone, sous forme de mélange d'isomères syn et anti, sépare les dits isomères syn et anti, puis saponifie séparément la fonction ester de chacun de ces isomères syn et anti au moyen d'une base forte, pour obtenir des produits de formule générale ($III_A$) sous forme d'isomère syn et sous forme d'isomère anti, dans laquelle $R_2$ représente un atome d'hydrogène, fixe sur la fonction acide de ces derniers, un groupement activateur de la fonction carbonyle, pour obtenir des produits de formule générale ($III_C$), sous forme d'isomères syn et anti, dans laquelle $R_2$ représente le groupement activateur précité, puis fait réagir ces derniers avec un acide aminé accepteur d'iode marqué à l'iode 125 ou 131 ou avec un dérivé d'un tel acide et obtient le produit cherché de formule générale (I) sous forme d'isomère syn et d'isomère anti séparés.

L'invention a notamment pour objet un procédé tel que défini précédemment, caractérisé en ce que l'acide aminé accepteur d'iode ou le dérivé d'acide aminé est choisi dans le groupe constitué par l'histidine, la tyrosine, l'histamine, la tyramine, ou le tyrosinate de méthyle ; l'halogénure de carboxyméthoxyl-amine est l'hémihydrochlorure de carboxyméthoxylamine et on opère sous atmosphère inerte, à l'obscurité et en présence de soude ; on fixe sur la fonction acide un groupement activateur de la fonction carbonyle en faisant agir un halogéno formiate d'alcoyle en présence d'une base tertiaire, en milieu anhydre et sous atmosphère inerte.

Il résulte de ce qui précède que le groupement activateur de la fonction carbonyle a la formule :

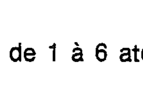

alk représentant un radical alkyle renfermant de 1 à 6 atomes de carbone.

Dans les conditions préférentielles de mise en œuvre, le procédé est caractérisé par les points suivants :

— l'halogéno formiate d'alcoyle est le chloroformiate d'isobutyle et on opère en présence de tri-n-butylamine ;

— l'agent d'estérification est le diazoéthane ;

— on saponifie la fonction ester au moyen de la soude méthanolique ;

— l'acide aminé ou son dérivé, que l'on fait réagir avec le produit de formule générale ($III_C$) dans laquelle $R_2$ représente un groupement activateur de la fonction carbonyle, est l'histamine marquée à l'iode 125 ou 131, et l'on opère sous atmosphère inerte et à l'obscurité.

L'activation de la fonction carbonyle du produit de formule générale ($III_A$) dans laquelle $R_2$ représente un atome d'hydrogène, peut aussi être réalisée en faisant agir sur celui-ci le N-hydroxy succinimide ou le dicyclohexylcarbodiimide, générateur de groupement activateur de ladite fonction carbonyle.

Le procédé décrit permet tout particulièrement d'obtenir au départ de la 17 β-hydroxy-4,9,11-trièn-3-one, le produit de formule :

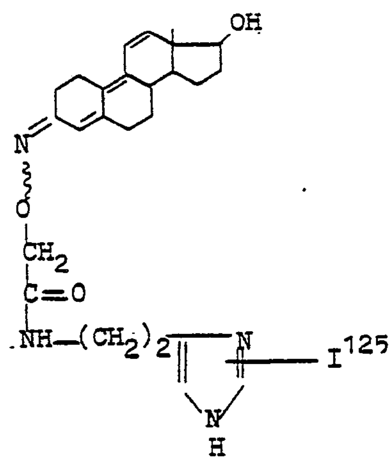

sous forme de mélange d'isomères syn et anti, ou sous forme d'isomère syn ou sous forme d'isomère anti, et au départ de la 17α-hydroxy-4,9,11-trièn-3-one, le produit de formule :

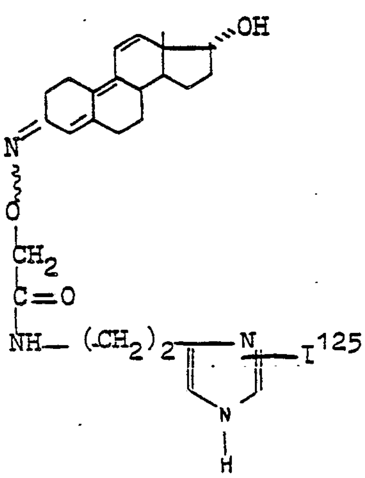

sous forme de mélange d'isomères syn et anti, ou sous forme d'isomère syn ou sous forme d'isomère anti.

L'invention a également pour objet l'application des produits de formule générale (I), objet de l'invention, aux dosages radioimmunologiques des 17β et 17α-hydroxy-4,9,11-trièn-3-ones dans la bile, l'urine, les fèces, le plasma des animaux ou de l'homme et les tissus des animaux, ainsi que dans les aliments des animaux ou de l'homme.

L'invention a également pour objet à titre de produits industriels nouveaux et notamment à titre de produits intermédiaires nécessaires à l'exécution du procédé de l'invention, les produits de formule générale (III) sous forme de mélange d'isomères syn et anti, ou sous forme d'isomère syn ou sous forme d'isomère anti :

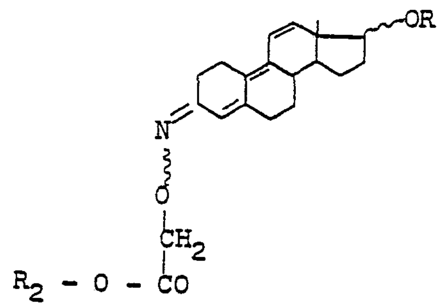

(III)

dans laquelle les lignes ondulées signifient que la fonction OR peut être en position α ou β et que la fonction oxime peut être en position d'isomère syn ou d'isomère anti et dans laquelle R représente un atome d'hydrogène, un groupement alcoyle ayant de 1 à 6 atomes de carbone ou un groupement acyle d'un acide carboxylique ayant de 2 à 12 atomes de carbone, $R_2$ représente un atome d'hydrogène, un groupement alcoyle ayant de 1 à 6 atomes de carbone ou un groupement activateur de la fonction carbonyle. Ce groupement est constitué par un radical de formule : —CO—O—alk, alk étant un radical alcoyle ayant de 1 à 6 atomes de carbone.

Parmi ces produits, on citera les produits suivants :

—17β-hydroxy-4,9,11-trièn-3-carboxyméthyloxime sous forme de mélange d'isomères syn et anti, sous forme d'isomère syn et sous forme d'isomère anti ;

—17α-hydroxy-4,9,11-trièn-3-carboxyméthyloxime sous forme de mélange d'isomères syn et anti, sous forme d'isomère syn et sous forme d'isomère anti ;

—17β-hydroxy-4,9,11-trièn-3-carbéthoxyméthyloxime sous forme de mélange d'isomères syn et anti, sous forme d'isomère syn et sous forme d'isomère anti ;

—17α-hydroxy-4,9,11-trièn-3-carbéthoxyméthyloxime sous forme de mélange d'isomères syn et anti, sous forme d'isomère syn et sous forme d'isomère anti ;

— l'anhydride mixte de 17β-hydroxy-4,9,11-trièn-3-carboxyméthyloxime avec le formiate d'isobutyle sous forme de mélange d'isomères syn et anti, sous forme d'isomère syn et sous forme d'isomère anti de formule :

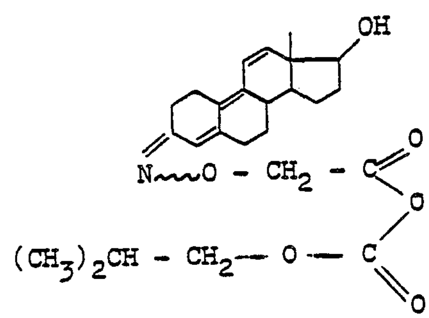

dans laquelle la ligne ondulée signifie que la fonction oxime peut être en position d'isomère syn, en position d'isomère anti ou peut représenter le mélange de ces deux isomères ;

— l'anhydride mixte de 17α-hydroxy-4,9,11-trièn-3-carboxyméthyloxime avec le formiate d'isobutyle sous forme de mélange d'isomères syn et anti, sous forme d'isomère syn et sous forme d'isomère anti, de formule :

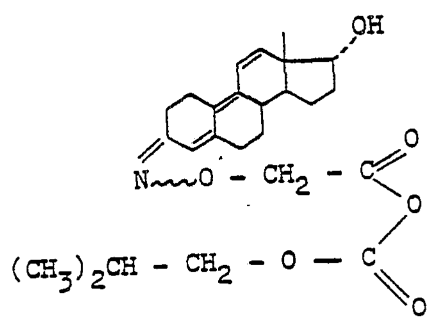

dans laquelle la ligne ondulée signifie que la fonction oxime peut être en position d'isomère syn, en position d'isomère anti ou peut représenter le mélange de ces deux isomères.

Les produits de formule générale (III), notamment ceux dans lesquels $R_2$ représente un atome d'hydrogène, sont des produits de départ utiles pour la préparation d'antigènes, nécessaires également aux dosages radioimmunologiques des 17β et 17α-hydroxy-4,9,11-trièn-3-one.

Leur application en tant que telle fait également l'objet de la présente invention et est caractérisée en ce que l'on conjugue un de ces produits avec l'albumine sérique bovine (BSA) ou avec l'albumine sérique humaine (HSA) et obtient l'antigène cherché.

Dans des conditions préférentielles de mise en œuvre, l'application des produits de formule générale (III), dans laquelle $R_2$ représente un atome d'hydrogène, est caractérisée par les points suivants :

— on fait réagir un produit de formule générale ($III_A$) sous forme de mélange d'isomères syn et anti, sous forme d'isomère syn ou sous forme d'isomère anti :

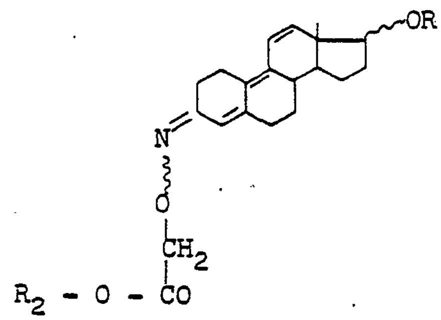

($III_A$)

dans laquelle les lignes ondulées et R ont les significations précitées et $R_2$ représente un atome d'hydrogène en vue de l'activation de la fonction carbonyle, avec un halogéno formiate d'alcoyle, en présence d'une base tertiaire, en milieu anhydre et sous atmosphère inerte ;

— on obtient l'anhydre mixte correspondant de formule ($III_C$) :

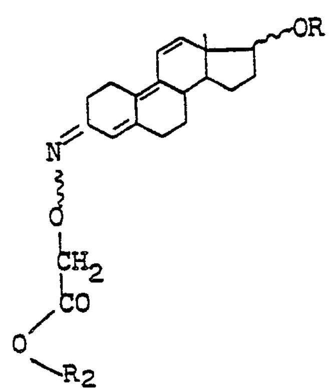

($III_C$)

dans laquelle $R_2$ représente un radical de formule —CO—O—alk, alk étant un radical alcoyle ayant au plus 6 atomes de carbone,

— que l'on conjugue avec l'albumine sérique bovine (BSA) ou humaine (HSA) et obtient l'antigène cherché.

Dans des conditions préférentielles de réalisation, l'application des produits de formule générale (III) ci-dessus est caractérisée par les points suivants :

— l'halogéno formiate d'alcoyle est le chloroformiate d'isobutyle et on opère en présence de tri-n-butylamine et sous atmosphère inerte ;

— on fait réagir l'anhydride mixte de formule générale ($III_C$) avec l'albumine sérique bovine (BSA) ou humaine (HSA) en ayant préalablement dissout cette dernière dans un mélange eau-dioxane sous atmosphère inerte.

L'application décrite permet d'obtenir respectivement au départ de :

— 17β-OR-4,9,11-trièn-3-carboxyméthyloxine, sous forme de mélange d'isomères syn et anti, sous forme d'isomère syn et sous forme d'isomère anti, les antigènes de formule :

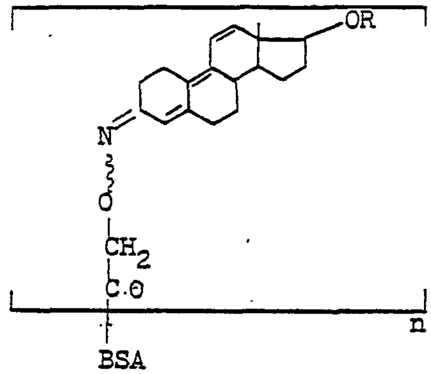

(IV)

dans laquelle n = 20 à 30 et la partie oxime de stéroïde est sous forme d'isomère syn, d'isomère anti ou sous forme de mélange d'isomères syn et anti :

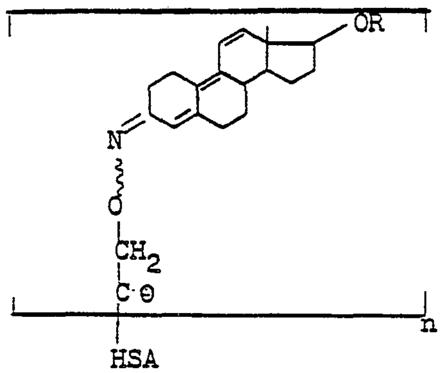

(V)

dans laquelle n = 20 à 30 et la partie oxime de stéroïde est sous forme d'isomère syn, d'isomère anti ou sous forme de mélange d'isomères syn et anti ;
et au départ de 17α-OR-4,9,11-trièn-3-carboxyméthyloxime sous forme de mélange d'isomères syn et anti, sous forme d'isomère syn et sous forme d'isomère anti, les antigènes de formule :

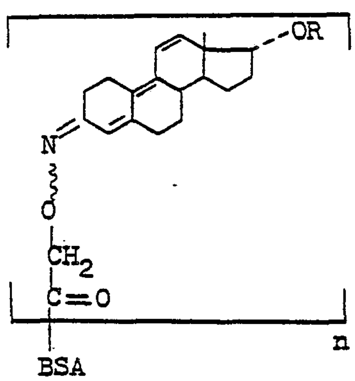

(VI)

dans laquelle n = 20 à 30 et la partie oxime de stéroïde est sous forme d'isomère syn, sous forme d'isomère anti ou sous forme de mélange d'isomère syn et anti :

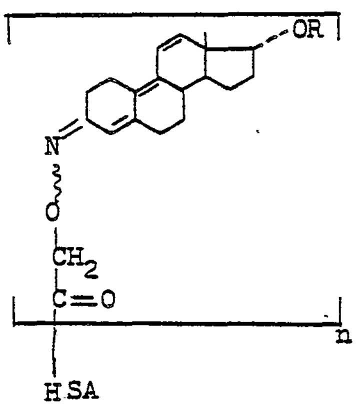

(VII)

dans laquelle n = 20 à 30 et la partie oxime de stéroïde est sous forme d'isomère syn, sous forme d'isomère anti ou sous forme de mélange d'isomères syn et anti.

L'invention a également pour objet, à titre de produits industriels nouveaux, les antigènes obtenus lors de l'application décrite ci-dessus, c'est-à-dire les produits de formules générales IV, V, VI et VII précitées.

L'invention a également pour objet l'application des antigènes ci-dessus à la préparation des anticorps.

Les produits de formule générale (I), objet de l'invention et notamment la 17β-hydroxy-4,9,11-trièn-3-carboyxméthyloxime ($^{125}I$) histamine sous forme d'isomère syn, sous forme d'isomère anti et sous forme de mélange d'isomères syn et anti, et la 17α-hydroxy-4,9,11-trièn-3-carboxyméthyloxime ($^{125}I$) histamine, sous forme d'isomère syn, sous forme d'isomère anti et sous forme de mélange d'isomères syn et anti, sont utilisés comme déjà dit lors de dosages radioimmunologiques des 17β et 17α-hydroxy-4,9,11-trièn-3-ones.

Ils permettent le dosage de ces produits dans les liquides et tissus biologiques, les fèces des animaux ou de l'homme ainsi que dans des produits d'alimentation humaine et animale.

Ils permettent notamment un dosage spécifique des quantités inférieures au p. p. b. de 17β et 17α-hydroxy-4,9,11-trièn-one, sans que l'on soit obligé de recourir aux méthodes d'isolement et de purification par chromatographie, avant de procéder au dosage de ces derniers.

Le dosage radioimmunologique a été effectué selon la méthode décrite par S. A. BERGSON et R. S. YALOW, HORMONE 4, p. 557 (1964) et G. E. ABRAHAM, Journal of Chemical Endocrinonal Metab. *29*, p. 866 (1969).

Les antigènes de formule IV, V, VI et VII précités, également objet de l'invention sont utilisés au développement d'anticorps injectés chez l'animal en présence d'un adjuvant, ils permettent, en effet, d'obtenir chez celui-ci des sérums contenant des anticorps contre le 17β-hydroxy-4,9,11-trièn-3-one et le 17α-hydroxy-4,9,11-trièn-3-one.

Les exemples suivants illustrent l'invention sans toutefois la limiter.

## Exemple 1

17β-hydroxy et 17α-hydroxy 4,9,11-trièn-3-carboxy-méthyloximes sous forme de mélange d'isomères syn et anti.

a) 17β-hydroxy 4,9,11-trièn-3-carboxyméthyloxime sous forme de mélange d'isomères syn et anti.

On prépare sous atmosphère inerte et dans l'obscurité une suspension de 3,29 g de 17β-hydroxy 4,9,11-trièn-3-one dans 85 ml d'éthanol puis ajoute successivement 3,05 g d'hémihydrochlorure de carboxyméthoxylamine et 15 ml de solution de NaOH N. On soumet le mélange réactionnel à température ambiante et à l'obscurité à l'agitation pendant 3 heures environ, le verse ensuite dans de l'eau glacée, en présence d'acide chlorhydrique, essore le précipité, le lave à l'eau jusqu'à neutralité des eaux de lavage et sèche sous vide. On obtient 3,86 g de produit cherché que l'on purifie par chromatographie sur silice. On élue par le mélange chlorure de méthylène, méthanol, acide acétique (90/10/1) et isole le produit de Rf = 0,35. Ce produit est un mélange d'isomères syn et anti.

Analyse : RMN à 90 MHz
529 Hz : $H_4$ de l'isomère anti
558-584 Hz : $H_4$ de l'isomère syn (masqué).

b) 17α-hydroxy 4,9,11-trièn-3-carboxyméthyloxime sous forme de mélange d'isomères syn et anti.

En procédant comme décrit ci-dessus, on prépare le produit à partir de 1,59 g de 17α-hydroxy 4,9,11-trièn-3-one, 40 ml d'éthanol, 1,5 g de hémihydrochlorure de carboxyméthoxylamine et 15 ml d'une solution de NaOH N. On obtient 1,75 g de produit cherché que l'on purifie par chromatographie sur silice. On élue par le mélange cyclohexane, acétate d'éthyle (70/30) et isole le produit de Rf = 0,25. Ce produit est un mélange d'isomères syn et anti.

## Exemple 2

Séparation d'isomères syn et anti des 17β-hydroxy et 17α-hydroxy 4,9,11-trièn-3-carboxyméthyloximes.

Stade A :

a) 17 β-hydroxy 4,9,11-trièn-3-carbéthoxyméthyloxime sous forme de mélange d'isomères syn et anti.

Estérification.

On prépare sous atmosphère inerte et sous agitation, une suspension de 1,3 g de 17β-hydroxy 4,9,11-trièn-3-carboxyméthyl-oxime, sous forme de mélange d'isomères syn et anti, dans 20 $cm^3$ de chlorure de méthylène, la refroidit vers 0 °C et l'additionne de 25 $cm^3$ de diazoéthane. On maintient le mélange réactionnel sous agitation et vers 0 °C pendant une heure environ, sépare la phase organique, l'évapore sous vide et obtient 1,53 g de produit cherché sous forme d'une huile, que l'on utilise telle quelle pour la séparation d'isomères syn et anti.

Séparation d'isomères.

On dissout le produit obtenu ci-dessus dans 3 $cm^3$ d'acétate d'éthyle, chromatographie la solution obtenue sur silice, on élue par le mélange cyclohexane, acétate d'éthyle (50/50). On obtient une première fraction contenant 750 mg d'un produit présumé être l'isomère anti, Rf = 0,32.

Une deuxième fraction constituée par 350 mg d'une huile est présumée comporter l'isomère syn. Rf = 0,28.

Les isomères séparés comme décrit ci-dessus seront saponifiés et analysés sous forme d'acides, afin de confirmer leurs structures syn et anti.

b) 17α-hydroxy 4,9,11-trièn-3-carbéthoxyméthyloxime sous forme de mélange d'isomères syn et anti.

En procédant comme décrit ci-dessus, on prépare, à partir de 1,64 g de 17α-hydroxy 4,9,11-trièn-3-carboxyméthyloxime, sous forme de mélange d'isomères syn et anti obtenu à l'exemple 1 a), 26 $cm^3$ de chlorure de méthylène et 35 $cm^3$ de diazoéthane, 1,55 g de 17α-hydroxy 4,9,11-trièn-3-carbéthoxyméthyloxime sous forme de mélange d'isomères syn et anti. La totalité de ce produit est chromatographiée sur silice avec élution par le mélange cyclohexane, acétate d'éthyle (70/30). On sépare une fraction A contenant 580 mg d'un produit présumé être l'isomère anti, Rf = 0,17. Une deuxième fraction B contient 490 mg d'un produit présumé être l'isomère syn, Rf = 0,13.

Ces deux produits seront saponifiés et analysés sous forme d'acides.

Stade B :

a) 17β-hydroxy 4,9,11-trièn-3-carboxyméthyloxime sous forme d'isomère syn.

On prépare sous atmosphère inerte et à l'obscurité, le mélange réactionnel de 350 mg de 17β-hydroxy 4,9,11-trièn-3-carbétoxyméthyloxime, isomère présumé syn, préparé au stade précédent, 5 $cm^3$ de méthanol et 1 $cm^3$ de lessive de soude, le soumet à température ambiante pendant une heure environ à l'agitation, puis refroidit vers 0 °C et additionne de 1 $cm^3$ d'acide chlorhydrique concentré. On essore le précipité formé, le sèche sous vide et obtient 295 mg de produit cherché.

Spectre RMN : 90 MHz

570-590 Hz : pour $H_4$, $H_{11}$ et $H_{12}$ d'isomère syn.

Spectre UV dans l'éthanol

— infl. 313 nm : $E_1^1$ = 883
— max. 325 nm : $E_1^1$ = 1144 ε = 39300
— infl. 335 nm : $E_1^1$ = 1098

Dichroïsme circulaire dans l'éthanol.

| | |
|---|---|
| — max. 231 nm | Δε = + 2,75 |
| — infl. 255 nm | Δε = − 2,25 |
| — infl. 290 nm | Δε = − 2,75 |
| — max. 302 nm | Δε = − 3,8 |
| — max. 315 nm | Δε = − 4,5 |
| — max. 331 nm | Δε = − 2,5 |
| — max. 347 nm | Δε = + 1,3 |

b) 17β-hydroxy 4,9,11-trièn-3-carboxyméthyloxime sous forme d'isomère anti.

En procédant comme pour l'isomère syn ci-dessus, on obtient à partir de 750 mg de 17β-hydroxy 4,9,11-trièn-3-carboxyméthyloxime, isomère présumé anti, obtenu selon le stade A de cet exemple, 10 $cm^3$ de méthanol et 1,6 $cm^3$ de lessive de soude. On obtient 660 mg de produit cherché.

Analyse :

Spectre RMN : 90 MHz.

526 Hz pour $H_4$ d'isomère anti.

Spectre UV dans l'éthanol.

— infl. 310 nm : $E_1^1$ = 855
— infl. 335 nm : $E_1^1$ = 1010 ε = 39300
— max. 322 nm : $E_1^1$ = 1144

Dichroïsme circulaire dans l'éthanol.

| | |
|---|---|
| — max. 231 nm | Δε = + 3,0 |
| — max. 253 nm | Δε = − 1,05 |
| — infl. 290 nm | Δε = − 2,7 |
| — max. 300 nm | Δε = − 4,2 |
| — max. 315 nm | Δε = − 4,6 |
| — max. 329 nm | Δε = − 1,6 |
| — max. 343 nm | Δε = + 2,75 |

c) 17α-hydroxy 3,4,11-trièn-3-carboxyméthyloxime isomère syn.

En procédant comme décrit ci-dessus, on obtient, à partir de 490 mg de 17α-hydroxy 3,4,11-trièn-3-carbétoxyméthyloxime isomère présumé syn, préparé selon le stade A de cet exemple, 6,5 $cm^3$ de méthanol et 1,1 $cm^3$ de lessive de soude, 325 mg de produit cherché.

Analyse

Spectre RMN : 90 MHz
532 Hz pour $H_2$
585 Hz pour $H_4$ de l'isomère syn.

Spectre UV dans l'éthanol/eau (1/4).

| | |
|---|---|
| Infl. 312 nm | $E^1_1$ = 888 |
| max. 325 nm | $E^1_1$ = 1 168 |
| infl. 335 nm | $E^1_1$ = 1 136 |

Dichroïsme circulaire dans éthanol/eau (1/4).

| | |
|---|---|
| max. 250 nm | Δε = — 3,3 |
| max. 303 nm | Δε = — 3,0 |
| max. 316 nm | Δε = — 3,1 |
| max. 350 nm | Δε = + 2,6 |

d) 17α-hydroxy 4,9,11-trièn-3-carboxyméthyloxime isomère anti.

En procédant comme décrit ci-dessus, on obtient, à partir de 580 mg de 17α-hydroxy 4,9,11-trièn-3-carbéthoxyméthyloxime isomère présumé anti, préparé selon le stade A de cet exemple, 7 $cm^3$ de méthanol et 1,3 $cm^3$ de lessive de soude, 440 mg de produit cherché.

Analyse :

Spectre RMN : 90 MHz
532 Hz pour $H_4$ d'isomère anti.

Spectre UV dans éthanol/eau (1/4).

| | |
|---|---|
| infl. 312 nm | $E^1_1$ = 874 |
| max. 323 nm | $E^1_1$ = 1 132 ε = 39 000 |
| infl. 335 nm | $E^1_1$ = 1 010 |

Dichroïsme circulaire dans éthanol/eau (1/4).

| | |
|---|---|
| max. 251 nm | $\Delta\varepsilon$ = — 2,4 |
| max. 303 nm | $\Delta\varepsilon$ = — 2,9 |
| max. 315 nm | $\Delta\varepsilon$ = — 2,8 |
| max. 349 nm | $\Delta\varepsilon$ = + 2,8 |

## Exemple 3

Antigènes à partir de 17β-hydroxy et 17α-hydroxy 4,9,11-trièn-3-carboxyméthyloxime isomères anti et d'albumine sérique bovine (BSA).

a) Antigène à partir de 17β-hydroxy 4,9,11-trièn-3-carboxyméthyloxime isomère anti.

Stade A : L'anhydride mixte de 17β-hydroxy 4,9,11-trièn-3-carboxyméthyloxime isomère anti avec le chloroformiate d'isobutyle.

On dissout sous agitation et sous atmosphère inerte 170 mg de 17β-hydroxy 4,9,11-trièn-3-carboxyméthyloxime isomère anti dans 5 $cm^3$ de dioxane, ajoute 0,23 $cm^3$ de tri-n-butylamine, puis abaisse la température vers 13 °C, ajoute 0,65 $cm^3$ de chloroformiate d'isobutyle et maintient l'agitation pendant 30 minutes à 13 °C environ. On obtient la solution de produit cherché que l'on utilise telle quelle pour le stade suivant.

Stade B : Dissolution de la BSA.

On introduit sous agitation et sous atmosphère inerte 690 mg de BSA dans 20 $cm^3$ d'eau glacée, puis ajoute après dissolution 2 $cm^3$ de dioxane, maintient la solution sous agitation environ 20 minutes, additionne successivement 20 $cm^3$ de dioxane et 0,7 $cm^3$ de NaOH N.

Stade C : Conjugaison.

On ajoute à cette solution de BSA la solution d'anhydride mixte obtenue précédemment au stade A, et soumet le mélange réactionnel à l'agitation pendant 4 heures à 4 °C environ.

On ajoute ensuite 50 $cm^3$ d'eau distillée, ajuste le pH à 4,1 par addition d'acide chlorhydrique N, sépare le précipité formé, puis le reprend par 50 $cm^3$ d'une solution aqueuse de bicarbonate de sodium à 1 %. La solution résultante est soumise à ultrafiltration, puis on fait passer 505 $cm^3$ d'eau afin d'épuiser les molécules de poids moléculaire < 10-15 000, et ajuste le volume final à 23 $cm^3$. On soumet cette solution à lyophilisation pendant 24 heures et obtient 759 mg de l'antigène cherché.

Analyse :

Spectre UV dans eau/présence de dioxane.

| | |
|---|---|
| max. 325 nm | $E^1_1$ = 125 |
| infl. 339 nm | $E^1_1$ = 98 |

Dichroïsme circulaire dans eau/présence de dioxane.

| | |
|---|---|
| max. 209 nm | $\Delta E^1_1$ = — 0,350 |
| max. 220 nm | $\Delta E^1_1$ = — 0,305 |
| max. 335 nm | $\Delta E^1_1$ = — 0,020 |

Ce produit contient 24 à 25 groupes de stéroïdes liés par mole.

b) Antigène à partir de 17α-hydroxy 4,9,11-trièn-3-carboxyméthyloxime isomère anti.

En procédant comme décrit sous a) de l'exemple 3 (stades A, B et C) en partant de 170 mg de 17α-hydroxy 4,9,11-trièn-3-carboxyméthyloxime isomère anti et de 690 mg de BSA, on obtient 912 mg d'antigène cherché. Ce produit contient 22 à 23 groupes de stéroïdes liés par mole.

Analyse :

Spectre UV dans eau/présence de dioxane.

| | |
|---|---|
| max. 325 nm | $E^1_1$ = 111 |
| infl. 340 nm | $E^1_1$ = 89 |

Dichroïsme circulaire.

| | |
|---|---|
| max. 213 nm | $\Delta E^1_1$ = — 0,328 |
| max. 223 nm | $\Delta E^1_1$ = — 0,324 |
| max. 305 nm | $\Delta E^1_1$ = — 0,010 |
| max. 316 nm | $\Delta E^1_1$ = — 0,009 |
| max. 351 nm | $\Delta E^1_1$ = + 0,011 |

## Exemple 4

Antigène à partir de 17α-hydroxy et 17β-hydroxy 4,9,11-trièn-3-carboxyméthyloxime, isomère syn et d'albumine sérique bovine (BSA).

a) Antigène à partir de 17α-hydroxy 4,9,11-trièn-3-carboxyméthyloxime, isomère syn.

En procédant comme décrit à l'exemple 3 (Stades A, B et C), en partant de 170 mg de 17α-hydroxy 4,9,11-trièn-3-carboxyméthyloxime isomère syn et de 690 mg de BSA, on obtient 902 mg d'antigène cherché. Ce produit contient 22 à 24 groupes de stéroïdes liés par mole.

Analyse :

Spectre UV dans eau/présence de dioxane.

| | |
|---|---|
| Max. 325 nm | $E^1_1$ = 118 |
| Infl. 340 nm | $E^1_1$ = 100 |

Dichroïsme circulaire.

| | |
|---|---|
| Max. 210 nm | $\Delta E^1_1$ = — 0,396 |
| Max. 222 nm | $\Delta E^1_1$ = — 0,010 |
| Max. 317 nm | $\Delta E^1_1$ = — 0,009 |
| Max. 351 nm | $\Delta E^1_1$ = + 0,012 |

b) Antigène à partir du 17β-hydroxy 4,9,11-trièn-3-carboxyméthyloxime, isomère syn.

On opère de manière analogue à celle décrite ci-dessus. On obtient le produit attendu.

De même, les antigènes peuvent d'une manière analogue être préparés à partir des mélanges d'isomères syn et anti, décrits dans l'exemple 1, des 17β-hydroxy et 17α-hydroxy-4,9,11-trièn-3-carboxyméthyloximes et de BSA.

En outre, de la même manière que celle décrite ci-dessus, on peut préparer également les antigènes par conjugaison de l'albumine sérique humaine (HSA) avec les mélanges d'isomères syn et anti des 17β-hydroxy et 17α-hydroxy 4,9,11-trièn-3-carboxyméthyl oximes, ainsi qu'avec chacun des isomères syn et anti isolés desdits composés stéroïdes, préparés selon les exemples 1 et 2.

## Exemple 5

17β-hydroxy 4,9,11-trièn-3-carboxyméthyloxime couplée à l'($^{125}$I), histamine sous forme de mélange d'isomères syn et anti, sous forme d'isomère syn et sous forme d'isomère anti.

Préparation d'anhydride mixte.

On dissout sous agitation et sous atmosphère inerte, 2,4 mg 17β-hydroxy 4,9,11-trièn-3-carboxyméthyloxime, sous forme de mélange d'isomères syn et anti dans 50 cm$^3$ de tétrahydrofuran, puis ajoute sous refroidissement 10 cm$^3$ de mélange tri-n-butylamine/tétrahydrofuran (1/5) et 10 cm$^3$ de mélange chloroformiate d'isobutyl/tétrahydrofuran (1/10).

Après une demi-heure d'agitation sous refroidissement, on ajoute 3,4 cm$^3$ de tétrahydrofuran. On obtient la solution d'anhydride mixte de 17β-hydroxy 4,9,11-trièn-3-carboxyméthyloxime sous forme de mélange d'isomères syn et anti, que l'on utilise tel quel pour le stade suivant.

Iodation de l'histamine.

A 10 µl d'une solution d'histamine 2 mM dans une solution tampon de phosphate de sodium 0,5 M de pH 8, on ajoute successivement 3,7 · $10^7$ Bq (1 mCi) de iodure $^{125}$ de sodium 7,4 · $10^{13}$ $s^{-1}$/mmol (2 000 Ci/mmol) et 50 µg de chloramine T dans 10 µl d'eau distillée, on agite le mélange réactionnel pendant 90 secondes environ, ajoute 300 µg de métabisulfite de sodium dissout dans 10 µl d'eau distillée et obtient

une solution aqueuse de produit cherché, Rf. = 0,1 (chromatographie sur silice, système de solvant méthanol/triéthylamine (98/2) que l'on utilise telle que pour la suite) :

Condensation :

On ajoute à cette solution d'histamine iodée, 50 µl d'anhydride mixte, précédemment préparée, agite et abandonne à l'obscurité sous refroidissement proche de 4 °C pendant une heure environ.

On dilue ensuite le mélange réactionnel dans 0,4 $cm^3$ de solution aqueuse de bicarbonate de sodium 0,1 M, extrait par 1,5 $cm^3$ de chlorure de méthylène, concentre la phase organique et obtient le produit cherché sous forme de mélange de ces isomères syn et anti.

On sépare les isomères par chromatographie liquide à haute performance avec élution par le mélange chloroforme/méthanol (97/3). On obtient un premier pic comportant le conjugué de l'isomère anti, de temps de rétention de 38 minutes et un deuxième pic comportant le conjugué de l'isomère syn, de temps de rétention de 42 minutes.

Le conjugué de l'isomère anti présente une activité totale de $5,55 \cdot 10^6$ Bq (150 µCi) et le conjugué de l'isomère syn présente une activité totale de $1,85 \cdot 10^6$ Bq (50 µCi).

Exemple 6

17α-hydroxy 4,9,11-trièn-3-carboxyméthyloxime couplée à l'($^{125}$I) histamine sous forme de mélange d'isomères syn et anti, sous forme d'isomère syn et sous forme d'isomère anti.

En procédant comme décrit dans l'exemple 5, on prépare au départ de 2,4 mg de 17α-hydroxy 4,9,11-trièn-3-carboxyméthyloxime, sous forme de mélange d'isomères syn et anti, dissout dans 50 $cm^3$ de tétrahydrofuran et de 10 $cm^3$ de mélange chloroformiate d'isobutyl/tétrahydrofuran (1/10), une solution d'anhydride mixte correspondant.

On prépare d'autre part comme décrit à l'exemple 5, une solution d'histamine iodée $^{125}$.

On condense comme décrit à l'exemple 5, 50 µl de solution d'anhydride mixte et la solution d'histamine iodée $^{125}$ ci-dessus et obtient le produit cherché sous forme de mélanges d'isomères syn et anti.

On sépare ces isomères par chromatographie liquide à haute performance (élution par le mélange chloroforme/méthanol (97/3).

On obtient un premier pic comportant le conjugué de l'isomère anti de temps de rétention de 34 minutes et un deuxième pic comportant le conjugué de l'isomère syn de temps de rétention de 42 minutes.

Le conjugué de l'isomère anti présente une activité totale de $1,85 \cdot 10^6$ Bq (50 µCi) et le conjugué de l'isomère syn présente une activité totale de $5,55 \cdot 10^6$ Bq (150 µCi).

**Revendications**

1. Produits estratriéniques marqués à l'iode$^{125}$ ou $^{131}$, sous forme d'isomères syn ou anti, ou sous forme de mélange d'isomères syn et anti, de formule générale (I) :

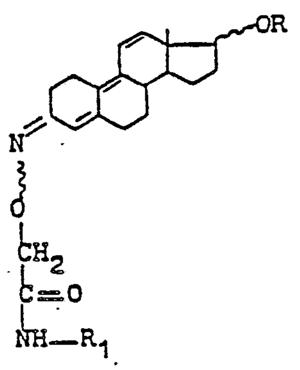

(I)

dans laquelle les lignes ondulées signifient que la fonction OR peut être en position α ou β et que la fonction oxime peut être en position syn ou anti, formule dans laquelle R représente un atome d'hydrogène, un groupement alcoyle ayant de 1 à 6 atomes de carbone ou un groupement acyle d'un acide carboxylique ayant de 2 à 12 atomes de carbone et $R_1$ représente le reste d'un acide aminé $R_1$ $NH_2$ ou le reste d'un dérivé de ce dernier, ce reste possédant un groupe accepteur d'iode et étant marqué à l'iode$^{125}$ ou $^{131}$.

2. Produits estratriéniques radioactifs marqués à l'iode de formule générale (I) selon la revendication 1, dans laquelle $R_1$ représente le reste d'un acide aminé $R_1$ $NH_2$ ou le reste d'un dérivé de ce dernier

choisi dans le groupe constitué par l'histidine, la tyrosine, l'histamine, la tyramine et le tyrosinate de méthyle marqués à l'iode$^{125}$ ou $^{131}$.

3. La 17β-hydroxy 4,9,11-trièn-3-carboxyméthyloxime sous forme d'isomère syn, couplée à l'($^{125}$I) histamine de formule

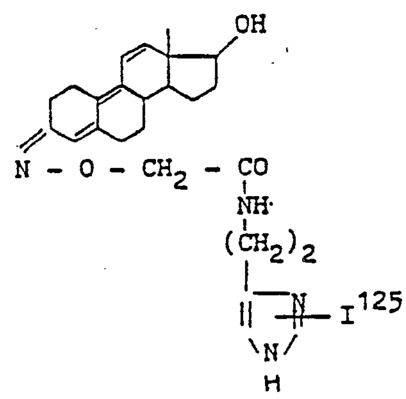

dans laquelle l'iode peut être en position 2 ou 5.

4. La 17β-hydroxy 4,9,11-trièn-3-carboxyméthyloxime sous forme d'isomère anti, couplée à l'($^{125}$I) histamine de formule

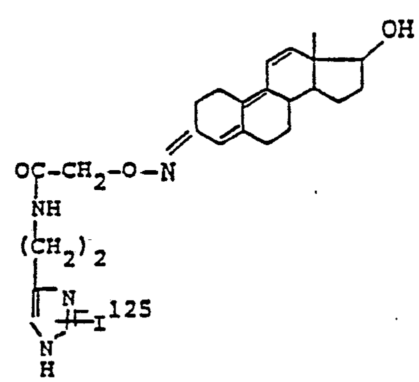

dans laquelle l'iode peut être en position 2 ou 5.

5. La 17β-hydroxy 4,9,11-trièn-3-carboxyméthyloxime sous forme d'isomère syn, couplée à l'($^{125}$I) histamine de formule

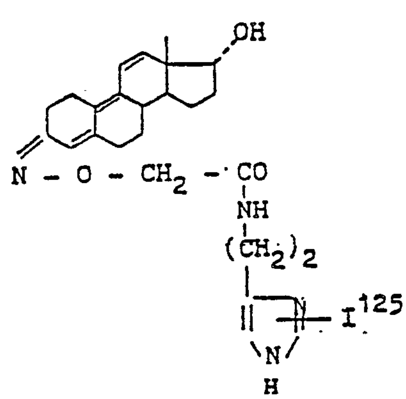

dans laquelle l'iode peut être en position 2 ou 5.

6. La 17α-hydroxy 4,9,11-trièn-3-carboxyméthyloxime sous forme d'isomère anti, couplée à l'($^{125}$I) histamine de formule

(Voir formule p. 16)

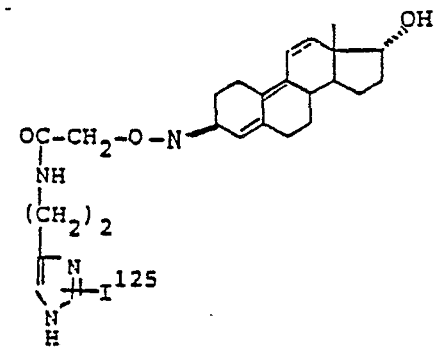

dans laquelle l'iode peut être en position 2 ou 5.

7. La 17β-hydroxy ou la 17α-hydroxy 4,9,11-trièn-3-carboxyméthyloxime sous forme de mélange d'isomères syn et anti couplées à l'($^{125}$I) histamine, l'iode pouvant être en position 2 ou 5.

8. Procédé de préparation des produits définis aux revendications 1 à 7, caractérisé en ce que

— on fait réagir un produit de formule générale (II) :

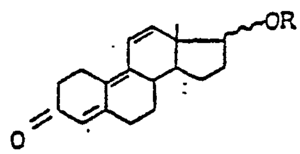

(II)

dans laquelle et dans ce qui suit R a les significations indiquées à la revendication 1, avec un halogénure de carboxyméthoxylamine en présence d'une base et obtient un produit de formule ($III_A$) :

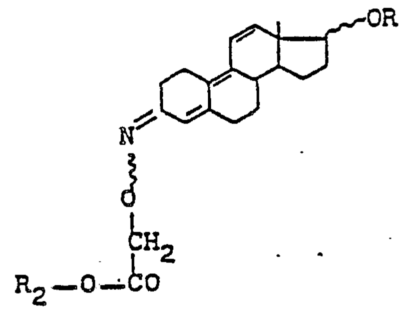

($III_A$)

dans laquelle la ligne ondulée sur l'azote indique que ce produit est sous forme de mélange d'isomères syn et anti et $R_2$ représente un atome d'hydrogène, puis

— soit fixe sur la fonction acide de ce produit un groupement activateur de la fonction carbonyle et obtient le produit de formule générale ($III_C$) :

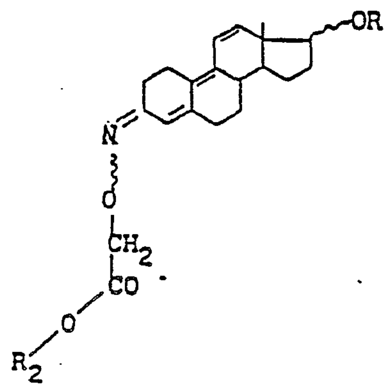

($III_C$)

dans laquelle $R_2$ représente un groupement activateur de la fonction carbonyle et la ligne ondulée sur l'azote indique que ce produit est sous forme de mélange d'isomères syn et anti, que l'on fait réagir avec un acide aminé accepteur d'iode marqué à l'iode$^{125}$ ou $^{131}$ ou avec un dérivé d'un tel acide et obtient le produit cherché de formule générale (I), que l'on isole sous forme de mélange d'isomères syn et anti, que l'on sépare éventuellement en ses isomères ;

— soit soumet le produit de formule générale ($III_A$), dans laquelle $R_2$ représente un atome d'hydrogène et la ligne ondulée sur l'azote indique que ce produit est sous forme de mélange d'isomères syn et anti, à l'action d'un agent d'estérification pour obtenir un produit de formule générale ($III_B$) :

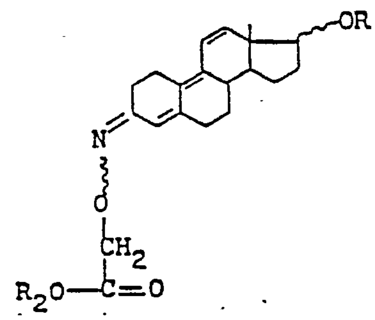

(III$_B$)

dans laquelle $R_2$ représente un groupement alcoyle ayant de 1 à 6 atomes de carbone, sous forme de mélange d'isomères syn et anti, sépare lesdits isomères syn et anti, puis saponifie séparément la fonction ester de chacun de ces isomères syn et anti au moyen d'une base forte, pour obtenir des produits de formule générale (III$_A$) sous forme d'isomère syn et sous forme d'isomère anti, dans laquelle $R_2$ représente un atome d'hydrogène, fixe sur la fonction acide de ces derniers un groupement activateur de la fonction carbonyle pour obtenir des produits de formule générale (III$_C$), sous forme d'isomère syn et sous forme d'isomère anti, dans laquelle $R_2$ représente le groupement activateur précité, puis fait réagir ces derniers avec un acide aminé accepteur d'iode marqué à l'iode$^{125}$ ou $^{131}$, ou avec un dérivé d'un tel acide et obtient le produit cherché de formule générale (I) sous forme d'isomère syn et d'isomère anti séparés.

9. Procédé selon la revendication 8, caractérisé en ce que l'acide aminé accepteur d'iode ou le dérivé d'acide aminé est choisi dans le groupe constitué par l'histidine, la tyrosine, l'histamine, la tyramine et le tyrosinate de méthyle.

10. Procédé selon la revendication 8 ou 9, caractérisé en ce que :

— l'halogénure de carboxyméthoxylamine est l'hémihydrochlorure de carboxyméthoxylamine et on opère sous atmosphère inerte, à l'obscurité et en présence de soude ;

— l'on fixe sur la fonction acide un groupement activateur de la fonction carbonyle en faisant agir un halogéno formiate d'alcoyle en présence d'une base tertiaire, en milieu anhydre et sous atmosphère inerte.

11. Procédé selon l'une quelconque des revendications 8 à 10, caractérisé en ce que :

— l'halogéno formiate d'alcoyle est le chloroformiate d'isobutyle et on opère en présence de tri-n-butylamine ;

— l'agent d'estérification est la diazoéthane ;

— l'on saponifie la fonction ester au moyen de la soude méthanolique,

— l'acide aminé ou son dérivé que l'on fait réagir avec le produit de formule générale III$_C$ dans laquelle $R_2$ représente un groupement activateur de la fonction carbonyle, est l'histamine marquée à l'iode 125 ou 131 et l'on opère sous atmosphère inerte et à l'obscurité.

12. Procédé suivant l'une quelconque des revendications 8 à 11, caractérisé en ce que le produit de départ étant la 17β-hydroxy 4,9,11-trièn-3-one, on obtient le produit de formule :

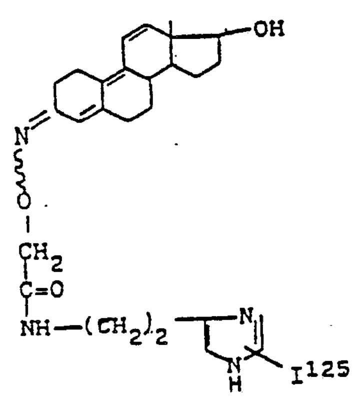

sous forme de mélange d'isomères syn et anti, ou sous forme d'isomère syn ou sous forme d'isomère anti.

13. Procédé suivant l'une quelconque des revendications 8 à 11, caractérisé en ce que le produit de départ étant la 17α-hydroxy 4,9,11-trièn-3-one, on obtient le produit de formule :

(Voir formule p. 18)

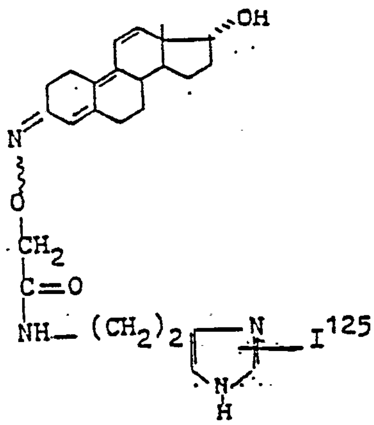

sous forme de mélange d'isomères syn et anti ou sous forme d'isomère syn ou sous forme d'isomère anti.

14. Application des produits tels que décrits à l'une quelconque des revendications 1 à 7, aux dosages radioimmunologiques de 17β et 17α-hydroxy 4,9,11-trièn-3-ones dans les liquides et les tissus biologiques et dans l'alimentation humaine et animale.

15. A titre de produits intermédiaires nécessaires pour la préparation des produits de formule générale I selon les revendicatidons 1 à 7, les produits de formule générale III sous forme de mélange d'isomères syn et anti, sous forme d'isomère syn ou sous forme d'isomère anti :

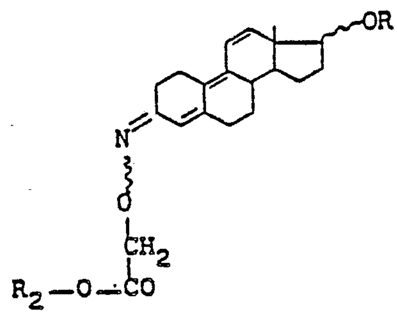

(III)

dans laquelle les lignes ondulées signifient que la fonction OR peut être en position α ou β et que la fonction oxime peut être en position syn ou anti et dans laquelle R représente un atome d'hydrogène, un groupement alcoyle ayant de 1 à 6 atomes de carbone ou un groupement acyle d'un acide carboxylique ayant de 2 à 12 atomes de carbone, $R_2$ représente un atome d'hydrogène, un groupement alcoyle ayant de 1 à 6 atomes de carbone ou un groupement activateur de la fonction carbonyle de formule —CO—O—alk, alk étant un radical alcoyle ayant de 1 à 6 atomes de carbone.

16. Les produits selon la revendication 15 dont les noms suivent :

— 17β-hydroxy 4,9,11-trièn-3-carboxyméthyloxime sous forme de mélange d'isomères syn et anti, sous forme d'isomères syn et sous forme d'isomère anti ;

— 17α-hydroxy 4,9,11-trièn-3-carboxyméthyloxime sous forme de mélange d'isomères syn et anti, sous forme d'isomère syn et sous forme d'isomère anti ;

— 17β-hydroxy 4,9,11-trièn-3-carbéthoxyméthyloxime sous forme de mélange d'isomères syn et anti, sous forme d'isomère syn et sous forme d'isomère anti ;

— 17α-hydroxy 4,9,11-trièn-3-carbéthoxyméthyloxime sous forme de mélange d'isomères syn et anti, sous forme d'isomère syn et sous forme d'isomère anti ;

— l'anhydride mixte de 17β-hydroxy 4,9,11-trièn-3-carboxyméthyloxime avec le formiate d'isobutyle sous forme de mélange d'isomères syn et anti, sous forme d'isomère syn et sous forme d'isomère anti :

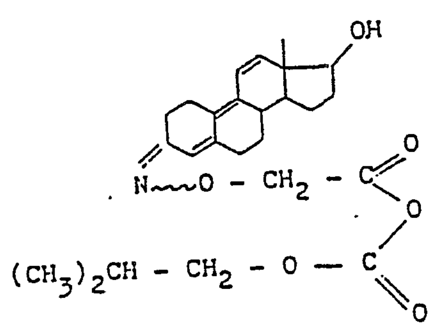

dans laquelle la ligne ondulée signifie que la fonction oxime peut être en position d'isomère syn, en position d'isomère anti ou peut représenter le mélange de ces deux isomères,

— l'anhydride mixte de 17α-hydroxy 4,9,11-trièn-3-carboxyméthyloxime avec le formiate d'isobutyle sous forme de mélange d'isomères syn et anti, sous forme d'isomère syn et sous forme d'isomère anti :

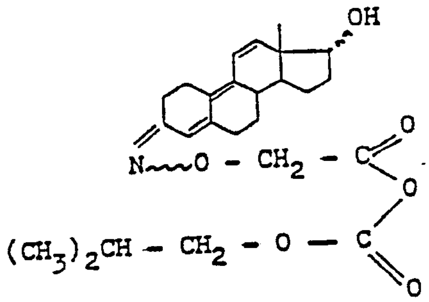

dans laquelle la ligne ondulée signifie que la fonction oxime peut être en position d'isomère syn, en position d'isomère anti, ou peut représenter le mélange de ces deux isomères.

17. Antigènes constitués par la conjugaison d'un produit de formule générale III selon la revendication 15, dans laquelle $R_2$ représente un atome d'hydrogène avec de l'albumine sérique bovine (BSA) ou avec l'albumine sérique humaine (HSA), à savoir les antigènes de formules :

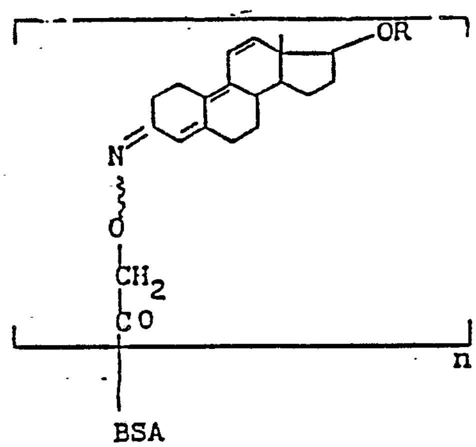

(IV)

dans laquelle n = 20 à 30 et la partie oxime de stéroïde est sous forme d'isomère syn, d'isomère anti et sous forme de mélange d'isomères syn et anti :

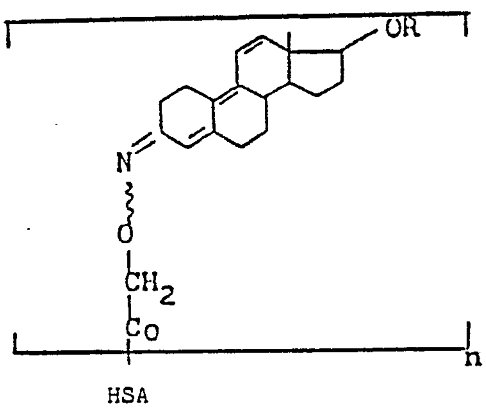

(V)

dans laquelle n = 20 à 30 et la partie oxime de stéroïde est sous forme d'isomère syn, sous forme d'isomère anti et sous forme de mélange d'isomères syn et anti :

(Voir formule p. 20)

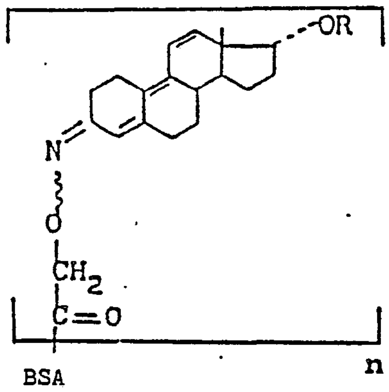

(VI)

dans laquelle n = 20 à 30 et la partie oxime de stéroïde est sous forme d'isomère syn, sous forme d'isomère anti et sous forme de mélange d'isomères syn et anti :

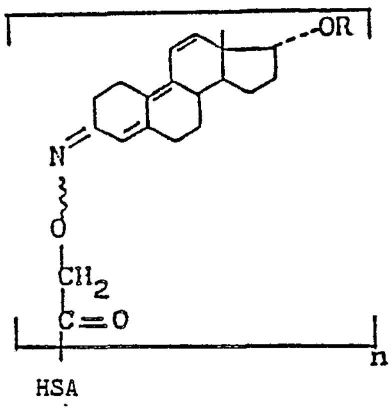

(VII)

dans laquelle n = 20 à 30 et la partie oxime de stéroïde est sous forme d'isomère syn, sous forme d'isomère anti et sous forme de mélange d'isomères syn et anti.

18. Procédé de préparation des antigènes décrits à la revendication 17 caractérisé en ce que l'on conjugue un produit de formule générale III telle que définie à la revendication 15 dans laquelle $R_2$ représente un atome d'hydrogène avec de l'albumine sérique bovine (BSA) ou avec de l'albumine sérique humaine (HSA) et obtient l'antigène recherché.

19. Procédé selon la revendication 18 caractérisé en ce que l'on fait réagir un produit de formule générale $III_A$ sous forme de mélange d'isomères syn et anti, sous forme d'isomère syn ou sous forme d'isomère anti :

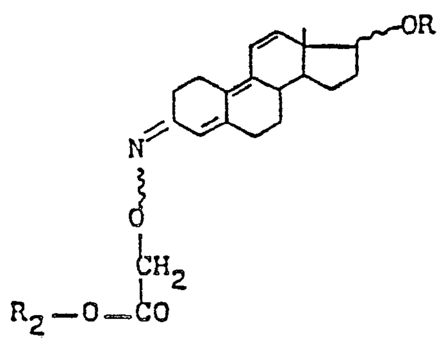

($III_A$)

dans laquelle les lignes ondulées et R ont les significations précitées et $R_2$ représente un atome d'hydrogène, en vue de l'activation de la fonction carbonyle, avec un halogénoformiate d'alcoyle, en présence d'une base tertiaire, en milieu anhydre et sous atmosphère inerte, obtient l'anhydride mixte correspondant, de formule $III_C$ :

(Voir formule p. 21)

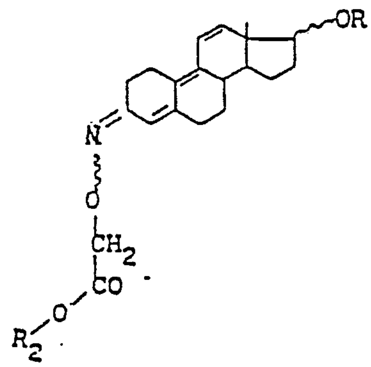

$(III_C)$

dans laquelle $R_2$ représente un radical de formule —CO—O—alc, alc étant un radical alcoyle ayant au plus 6 atomes de carbone, que l'on conjugue avec l'albumine sérique bovine (BSA) ou humaine (HSA) et obtient l'antigène cherché.

20. Procédé selon la revendication 19, caractérisé en ce que :

— l'halogénoformiate d'alcoyle est le chloroformiate d'isobutyle et l'on opère en présence de tri-n-butylamine et sous atmosphère inerte ;

— on fait réagir l'anhydride mixte de formule générale $III_C$ avec l'albumine sérique bovine (BSA) ou humaine (HSA) en ayant préalablement dissous cette dernière dans un mélange eau-dioxane sous atmosphère inerte.

21. Application des antigènes selon la revendication 17, à la préparation des anticorps.

**Claims**

1. Estratriene products labelled with iodine$^{125}$ or $^{131}$, in the form of syn or anti isomers, or in the form of a mixture of syn and anti isomers, with the general formula (I) :

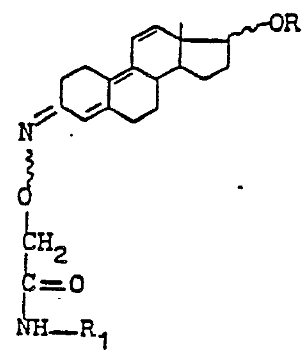

(I)

in which the wavy lines signify that the OR function can be in α or β position and that the oxime function can be in syn or anti position, in which formula R represents a hydrogen atom, an alkyl group having from 1 to 6 carbon atoms or an acyl group of a carboxylic acid having from 2 to 12 carbon atoms and $R_1$ represents the residue of an amino acid $R_1$ $NH_2$ or the residue of a derivative of this latter, this residue possessing an iodine acceptor group and being labelled with iodine$^{125}$ or $^{131}$.

2. Radio-active estratriene products marked with iodine with the general formula (I) according to Claim 1, in which $R_1$ represents the residue of an amino acid $R_1$ $NH_2$ or the residue of a derivative of this latter chosen from the group constituted by histidine, tyrosine, histamine, tyramine and methyl tyrosinate labelled with iodine$^{125}$ or $^{131}$.

3. 17β-hydroxy-4,9,11-trien-3-carboxymethyloxime in the form of the syn isomer, coupled to ($^{125}$I) histamine with the formula

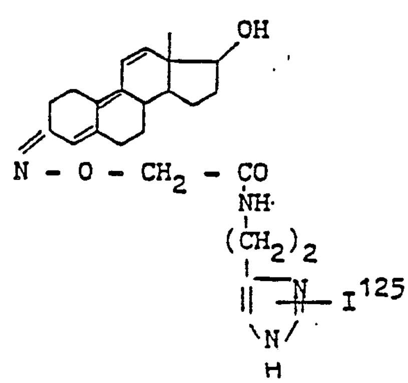

in which the iodine can be in position 2 or 5.

4. 17β-hydroxy-4,9,11-trien-3-carboxymethyloxime in the form of the anti isomer, coupled to ($^{125}$I) histamine with the formula

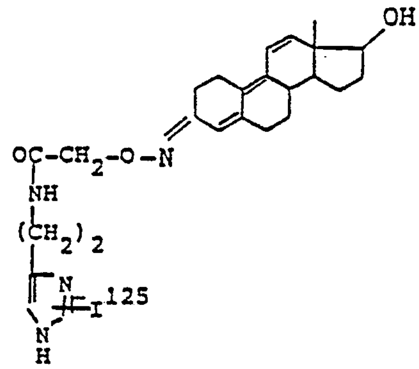

in which the iodine can be in position 2 or 5.

5. 17α-hydroxy-4,9,11-trien-3-carboxymethyloxime in the form of the syn isomer, coupled to ($^{125}$I) histamine with the formula

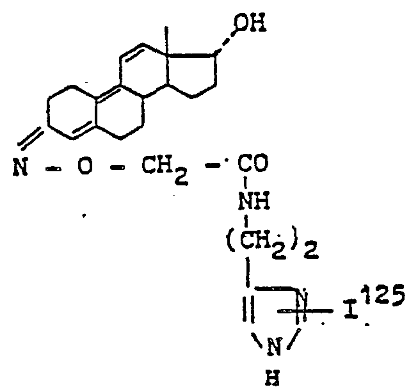

in which the iodine can be in position 2 or 5.

6. 17α-hydroxy-4,9,11-trien-3-carboxymethyloxime in the form of the anti isomer, coupled to ($^{125}$I) histamine with the formula

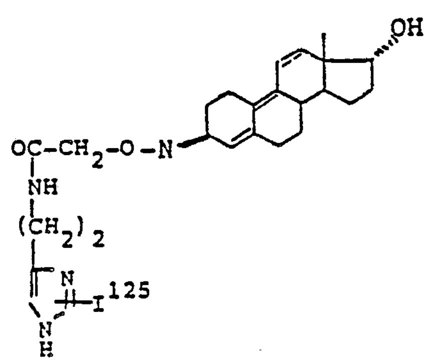

in which the iodine can be in position 2 or 5.

7. 17β-hydroxy or 17α-hydroxy-4,9,11-trien-3-carboxymethyloxime in the form of a mixture of syn and anti isomers coupled to ($^{125}$I) histamine, the iodine being able to be in position 2 or 5.

8. Process for the preparation of the products defined in Claims 1 to 7, characterized in that

— a product with the general formula (II) :

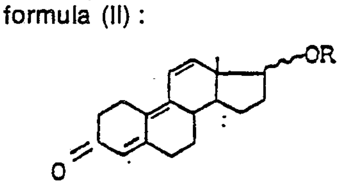

(II)

in which and in what follows R has the significances indicated in Claim 1, is made to react with a carboxymethoxylamine halogenide in the presence of a base and a product is obtained with the formula ($III_A$) .

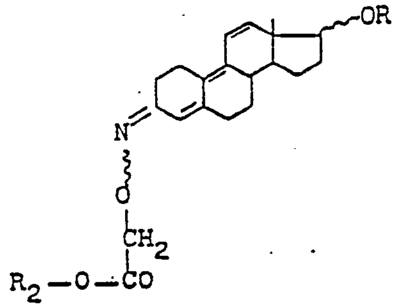

($III_A$)

in which the wavy line on the nitrogen indicates that this product is in the form of a mixture of syn and anti isomers and $R_2$ represents a hydrogen atom, then

— either an activator group of the carbonyl function is fixed on the acid function of this product and the product is obtained with the general formula ($III_C$).

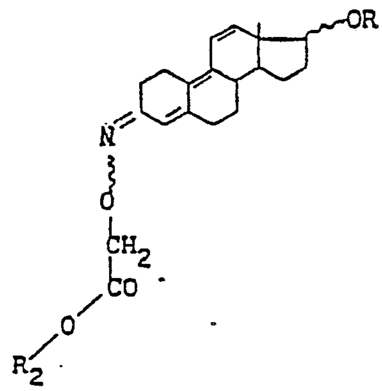

($III_C$)

in which $R_2$ represents an activator group of the carbonyl function and the wavy line on the nitrogen indicates that this product is in the form of a mixture of syn and anti isomers which is made to react with an iodine acceptor amino acid labelled with $iodine^{125}$ or $^{131}$ or with a derivative of such an acid and the product sought with the general formula (I) is obtained, which is isolated in the form of a mixture of syn and anti isomers, which can be separated into its isomers ;

— or the product with the general formula ($III_A$) in which $R_2$ represents a hydrogen atom and the wavy line on the nitrogen indicates that the product is in the form of a mixture of syn and anti isomers, is submitted to the action of an esterification agent in order to obtain a product with the general formula ($III_B$) :

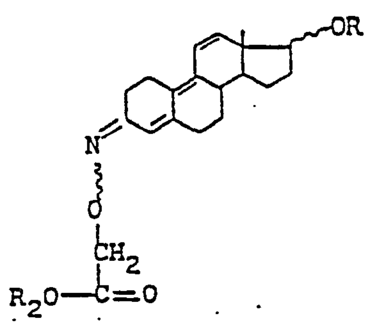

($III_B$)

in which $R_2$ represents an alkyl group having from 1 to 6 carbon atoms, in the form of a mixture of syn and anti isomers, the said syn and anti isomers are separated, then the ester function of each of these syn and anti isomers is saponified separately by means of a strong base, so as to obtain the products with the general formula ($III_A$) in the form of the syn isomer and in the form of the anti isomer, in which $R_2$ represents a hydrogen atom, an activator group of the carbonyl function is fixed on the acid function of these latter so as to obtain products with the general formula ($III_C$) in the form of the syn and in the form of the anti isomer, in which $R_2$ represents the aforementioned activator group, then these latter are made to react with an iodine acceptor amino acid labelled with $iodine^{125}$ or $^{131}$ or with a derivative of such an acid and the product sought with the general formula (I) is obtained in the form of the separated syn and anti isomers.

9. Process according to Claim 8, characterized in that the iodine acceptor amino acid or the

derivative of the amino acid is chosen from the group constituted by histidine, tyrosine, histamine, tyramine and methyl tyrosinate.

10. Process according to Claim 8 or 9, characterized in that :

— the carboxymethoxylamine halogenide is carboxymethoxylamine hemihydrochloride and the operation is done under an inert atmosphere, in the dark, and in the presence of sodium hydroxide ;

— an activator group of the carbonyl function is fixed on the acid function by making an alkyl halogeno formate act in the presence of a tertiary base in an anhydrous medium and under an inert atmosphere.

11. Process according to any one of the Claims 8 to 10, characterized in that.

— the alkyl halogeno formate is isobutyl chloroformate, and the operation is done in the presence of tri-n-butylamine :

— the esterification agent is diazoethane ;

— the ester function is saponified by means of methanol sodium hydroxide,

— the amino acid or its derivative which is made to react with the product with the general formula $III_C$ in which $R_2$ represents an activator group of the carbonyl function, is histamine labelled with iodine$^{125}$ or $^{131}$, and the operation is done under an inert atmosphere and in darkness.

12. Process according to any one of the Claims 8 to 11, characterized in that the starting product being 17β-hydroxy-4,9,11-trien-3-one, the product with the formula :

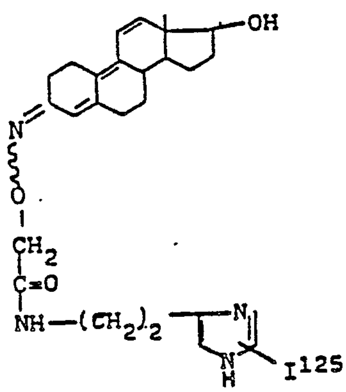

is obtained in the form of a mixture of syn and anti isomers or in the form of the syn isomer or in the form of the anti isomer.

13. Process according to any one of the Claims 8 to 11, characterized in that the starting product being 17α-hydroxy-4,9,11-trien-3-one, the product with the formula

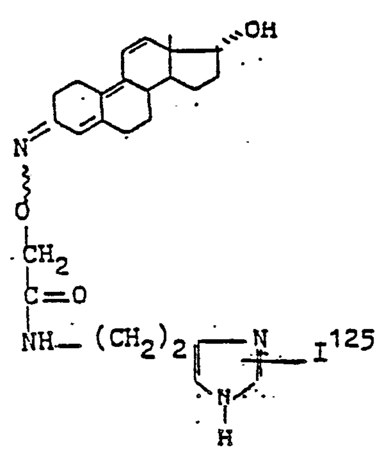

is obtained in the form of a mixture of syn and anti isomers or in the form of the syn isomer or in the form of the anti isomer.

14. Use of the products as described in any one of the Claims 1 to 7, at the radio-immunological dosages of 17β and 17α-hydroxy-4,9,11-trien-3-one in the biological liquids and tissues and in human and animal alimentation.

15. As intermediate products necessary for the preparation of the products with the general formula I according to Claims 1 to 7, the products with the general formula III in the form of a mixture of syn and anti isomers, in the form of syn isomer or in the form of anti isomer :

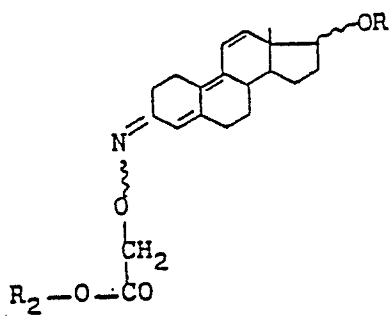

(III)

in which the wavy lines signify that the OR function can be in α or β position and that the oxime function can be in the syn or anti position and in which R represents a hydrogen atom, an alkyl group having form.1 to 6 carbon atoms or an acyl group of a carboxylic acid having from 2 to 12 carbon atoms, $R_2$ represents a hydrogen atom, an alkyl group having from 1 to 6 carbon atoms or an activator group of the carbonyl function with formula —CO—O—alk, alk being an alkyl radical having from 1 to 6 carbon atoms.

16. The products according to Claim 15, the names of which follow :

— 17β-hydroxy-4,9,11-trien-3-carboxymethyloxime in the form of a mixture of syn and anti isomers, in the form of the syn isomer and in the form of the anti isomer :

— 17α-hydroxy-4,9,11-trien-3-carboxymethyloxime in the form of a mixture of syn and anti isomers, in the form of the syn isomer and in the form of the anti isomer :

— 17β-hydroxy-4,9,11-trien-3-carbethoxymethyloxime in the form of a mixture of syn and anti isomers, in the form of the syn isomer and in the form of the anti isomer :

— 17α-hydroxy-4,9,11-trien-3-carbethoxymethyloxime in the form of a mixture of syn and anti isomers, in the form of the syn isomer and in the form of the anti isomer :

— the mixed anhydride of 17β-hydroxy-4,9,11-trien-3-carboxymethyl-oxime with isobutyl formate in the form of a mixture of syn and anti isomers, in the form of the syn isomer and in the form of the anti isomer :

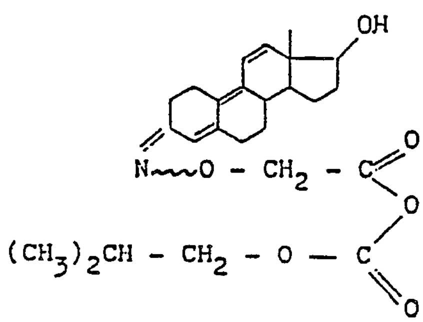

in which the wavy line signifies that the oxime function can be in the syn isomer position, in the anti isomer position, or can represent the mixture of these two isomers,

— the mixed anhydride of 17α-hydroxy-4,9,11-trien-3-carboxymethyloxime with isobutyl formate in the form of a mixture of syn and anti isomers, in the form of the syn isomer and in the form of the anti isomer :

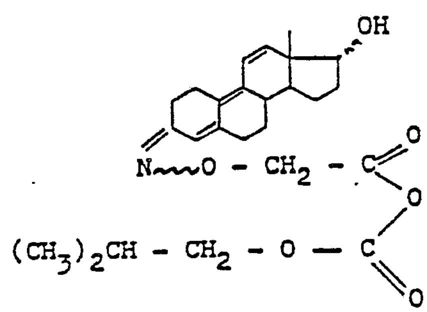

in which the wavy line signifies that the oxime function can be in the syn isomer position, in the anti isomer position, or can represent the mixture of these two isomers.

17. Antigens constituted by the conjugation of a product with the general formula III according to Claim 15, in which $R_2$ represents a hydrogen atom, with bovine seric albumin (BSA) or with human seric albumin (HSA), that is to say, the antigens with the formulae

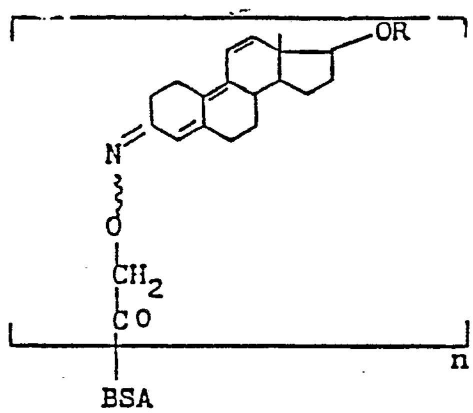

(IV)

in which n = 20 to 30 and the oxime part of the steroid is in the form of the syn isomer, of the anti isomer, and in the form of a mixture of syn and anti isomers :

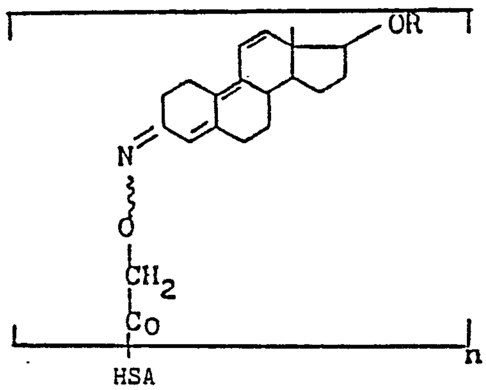

(V)

in which n = 20 to 30 and the oxime part of the steroid is in the form of the syn isomer, of the anti isomer, and in the form of a mixture of syn and anti isomers :

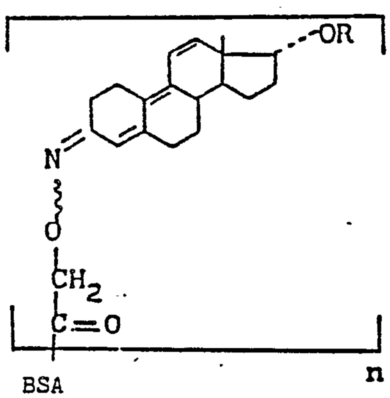

(VI)

in which n = 20 to 30 and the oxime part of the steroid is in the form of the syn isomer, in the form of the anti isomer, and in the form of a mixture of syn and anti isomers :

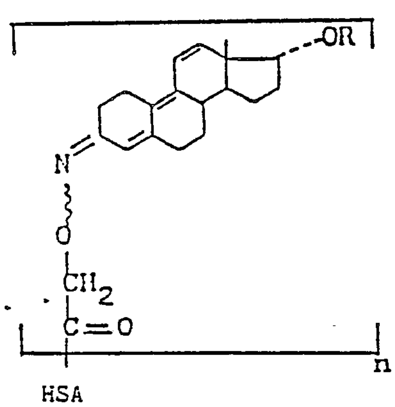

(VII)

in which n = 20 to 30 and the oxime part of the steroid is in the form of the syn isomer, in the form of the anti isomer, and in the form of a mixture of syn and anti isomers.

18. Preparation process for the antigens described in Claim 17, characterized in that a product with the general formula III as defined in Claim 15 in which $R_2$ represents a hydrogen atom is conjugated with bovine seric albumin (BSA) or with human seric albumin (HSA) and the antigen sought is obtained.

19. Process according to Claim 18 characterized in that a product with the general formula $III_A$ in the form of a mixture of syn and anti isomers, in the form of the syn isomer or in the form of the anti isomer :

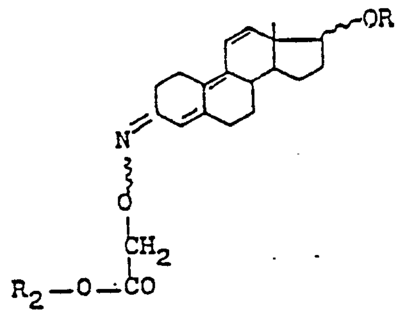

($III_A$)

in which the wavy lines and R have the significances previously stated and $R_2$ represents a hydrogen atom, is made to react, with a view to the activation of the carbonyl function, with an alkyl halogenoformate, in the presence of a tertiary base, in an anhydrous medium, and under an inert atmosphere, the corresponding mixed anhydride with the formula $III_C$ :

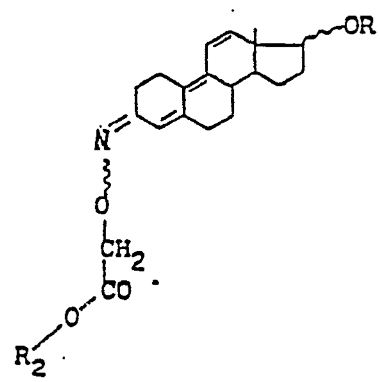

($III_C$)

is obtained, in which $R_2$ represents a radical with the formula —CO—O—alk, alk being an alkyl radical having at the most 6 carbon atoms, which is conjugated with bovine seric albumin (BSA) or with human seric albumin (HSA), and the antigen sought is obtained.

20. Process according to Claim 19, characterized in that :

— the alkyl halogenoformate is isobutyl chloroformate, and the operation is done in the presence of tri-n-butylamine and under an inert atmosphere :

— the mixed anhydride with the general formula $III_C$ is made to react with bovine seric albumin (BSA) or with human seric albumin (HSA) after having previously dissolved this latter in a water-dioxan mixture under an inert atmosphere.

21. Use of the antigens according to Claim 17, for the preparation of the anti-bodies.

**Patentansprüche**

1. Jod$^{125}$- oder -$^{131}$-markierte Östratienprodukte in Form der syn- oder anti-Isomeren oder in Form eines Gemisches der syn- und anti-Isomeren der allgemeinen Formel (I)

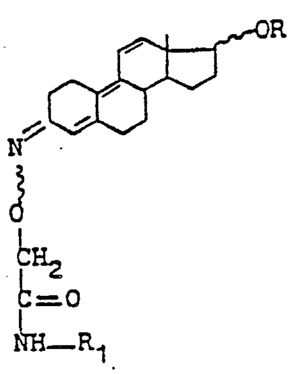

(I)

worin die gewellten Linien anzeigen, daß die OR-Funktion sich in α- oder β-Stellung befinden kann und daß die Oxim-Funktion sich in syn- oder anti-Stellung befinden kann, R ein Wasserstoffatom, eine Alkylgruppe mit 1 bis 6 Kohlenstoffatomen oder eine Acylgruppe einer Carbonsäure mit 2 bis 12 Kohlenstoffatomen bedeutet und $R_1$ den Rest einer Aminosäure $R_1NH_2$ oder den Rest eines Derivats derselben darstellt, wobei dieser Rest eine Jodakzeptorgruppe besitzt und $Jod^{125}$- oder $-^{131}$-markiert ist.

2. Jod-markierte, radioaktive Östratrienprodukte der allgemeinen Formel (I) gemäß Anspruch 1, worin $R_1$ den $Jod^{125}$- oder $-^{131}$-markierten Rest einer Aminosäure $R_1NH_2$ oder den $Jod^{125}$- oder $-^{131}$-markierten Rest eines Derivates derselben, ausgewählt unter Histidin, Tyrosin, Histamin, Tyramin und Methyltyrosinat, bedeutet.

3. Mit ($^{125}$I)-Histamin gekuppeltes 17β-Hydroxy-4,9,11-trien-3-carboxymethyloxim in Form des syn-Isomeren der Formel

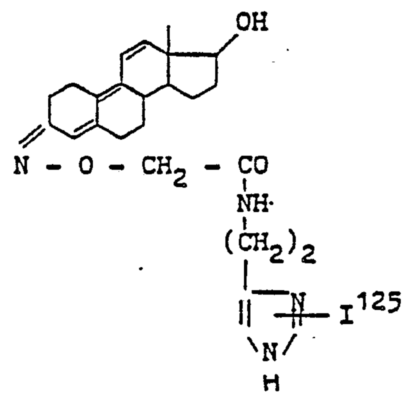

worin sich das Jod in 2- oder 5-Stellung befinden kann.

4. Mit ($^{125}$I)-Histamin gekuppeltes 17-Hydroxy-4,9,11-trien-3-carboxymethyloxim in Form des anti-Isomeren der Formel

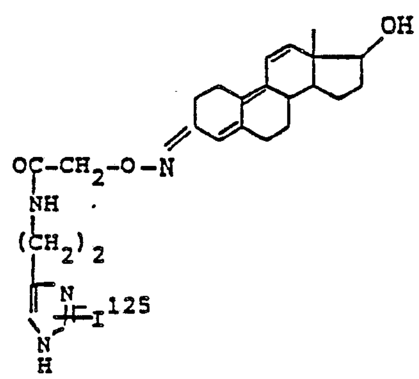

worin sich das Jod in 2- oder 5-Stellung befinden kann.

5. Mit ($^{125}$I)-Histamin gekuppeltes 17α-Hydroxy-4,9,11-trien-3-carboxymethyloxim in Form des syn-Isomeren der Formel

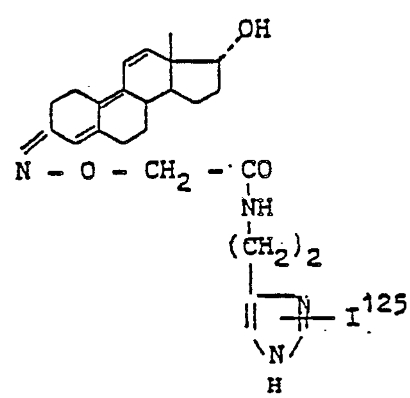

worin sich das Jod in 2- oder 5-Stellung befinden kann.

6. Mit ($^{125}$I)-Histamin gekuppeltes 17α-Hydroxy-4,9-11-trien-3-carboxymethyloxim in Form des anti-Isomeren der Formel

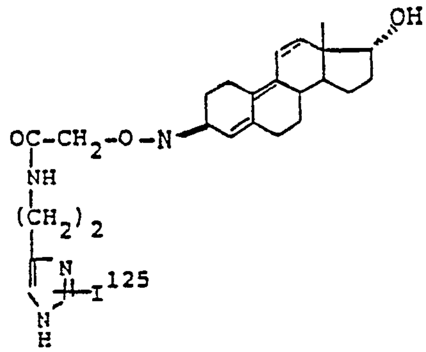

worin sich das Jod in 2- oder 5-Stellung befinden kann.

7. Mit ($^{125}$I)-Histamin gekuppeltes 17β-Hydroxy- oder 17α-Hydroxy-4,9,11-trien-3-carboxymethyloxim in Form eines Gemisches der syn- und anti-Isomeren, worin sich das Jod in 2- oder 5-Stellung befinden kann.

8. Verfahren zur Herstellung der Produkte gemäß den Ansprüchen 1 bis 7, dadurch gekennzeichnet, daß

— man ein Produkt der allgemeinen Formel (II)

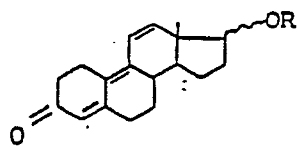

(II)

in der und im folgenden R die in Anspruch 1 angegebenen Bedeutungen besitzt, mit einem Carboxymethoxylamin-halogenid in Anwesenheit einer Base umsetzt und ein Produkt der Formel ($III_A$)

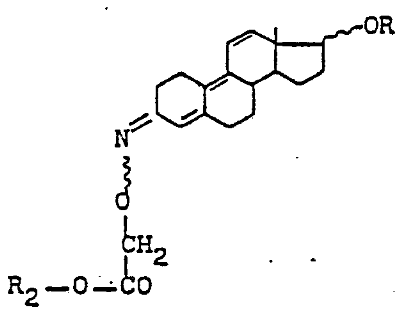

($III_A$)

gewinnt, worin die gewellte Linie an dem Stickstoff anzeigt, daß das Produkt in Form eines Gemisches der syn- und anti-Isomeren vorliegt und $R_2$ ein Wasserstoffatom bedeutet, danach

— entweder an der Säurefunktion dieses Produkts eine Aktivatorgruppe für die Carbonylfunktion fixiert und das Produkt der allgemeinen Formel ($III_C$)

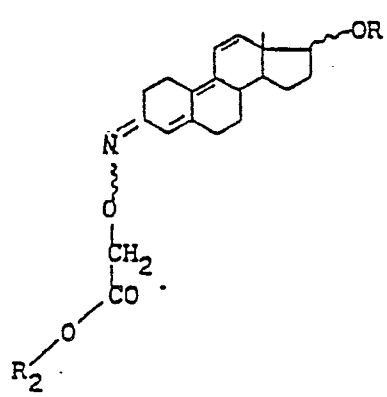

($III_C$)

worin $R_2$ eine Aktivatorgruppe für die Carbonylfunktion bedeutet und die gewellte Linie an dem Stickstoff anzeigt, daß das Produkt in Form eines Gemisches der syn- und anti-Isomeren vorliegt, erhält, welches man mit einem Jod$^{125}$- oder -$^{131}$-markierten Aminosäure-Jodakzeptor oder mit einem Derivat einer derartigen Säure umsetzt und das gewünschte Produkt der allgemeinen Formel (I) erhält, das man in Form eines Gemisches des syn- und anti-Isomeren isoliert, welches man gegebenenfalls in seine Isomeren auftrennt,

— oder das Produkt der allgemeinen Formel ($III_A$), worin $R_2$ ein Wasserstoffatom bedeutet und die gewellte Linie an dem Stickstoff anzeigt, daß dieses Produkt in Form eines Gemisches der syn- und anti-Isomeren vorliegt, der Einwirkung eines Veresterungsmittels unterzieht, um ein Produkt der allgemeinen Formel ($III_B$)

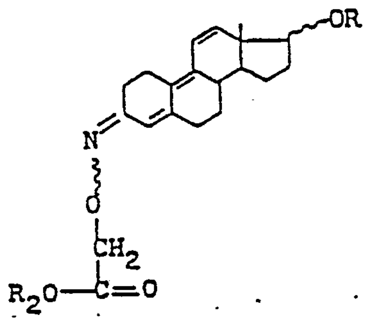

($III_B$)

worin $R_2$ eine Alkylgruppe mit 1 bis 6 Kohlenstoffatomen bedeutet, in Form eines Gemisches der syn- und anti-Isomeren zu erhalten, diese syn- und anti-Isomeren auftrennt, danach getrennt die Esterfunktion eines jeden dieser syn- und anti-Isomeren mit einer starken Base verseift, um Produkte der allgemeinen Formel ($III_A$) in Form des syn-Isomeren und in Form des anti-Isomeren, worin $R_2$ ein Wasserstoffatom bedeutet, zu erhalten, an der Säurefunktion dieser letzteren eine Aktivatorgruppe für die Carbonylfunktion fixiert, um Produkte der allgemeinen Formel ($III_C$) in Form des syn-Isomeren und in Form des anti-Isomeren, worin $R_2$ die vorstehende Aktivatorgruppe darstellt, zu erhalten, danach diese letzteren mit einem Jod$^{125}$- oder -$^{131}$-markierten Aminosäure-Jodakzeptor oder mit einem Derivat einer derartigen Säure umsetzt, und das gewünschte Produkt der allgemeinen Formel (I) getrennt in Form des syn-Isomeren und des anti-Isomeren zu erhalten.

9. Verfahren gemäß Anspruch 8, dadurch gekennzeichnet, daß der Aminosäure-Jodakzeptor oder das Derivat dieser Aminosäure unter Histidin, Tyrosin, Histamin, Tyramin und Methyltyrosinat ausgewählt wird.

10. Verfahren gemäß Anspruch 8 oder 9, dadurch gekennzeichnet, daß

— das Carboxymethoxylamin-halogenid Carboxymethoxylamin-hemihydrochlorid ist und daß man unter inerter Atmosphäre im Dunkeln und in Anwesenheit von Natronlauge arbeitet.

— man an das Säurefunktion eine Aktivatorgruppe für die Carbonylfunktion fixiert, indem man mit einem Alkylhalogenoformiat in Anwesenheit einer tertiären Base in wasserfreiem Milieu und unter inerter Atmosphäre umsetzt.

11. Verfahren gemäß einem der Ansprüche 8 bis 10, dadurch gekennzeichnet, daß

— das Alkylhalogenoformiat Isobutylchloroformiat ist und daß man in Anwesenheit von Tri-n-butylamin arbeitet,

— das Veresterungsmittel Diazoethan ist,

— man die Esterfunktion mit Hilfe von methanolischer Natronlauge verseift,

— die Aminosäure oder ihr Derivat, welche man mit dem Produkt der allgemeinen Formel ($III_C$), worin $R_2$ eine Aktivatorgruppe für die Carbonylfunktion bedeutet, umsetzt, J$^{125}$- oder -$^{131}$-markiertes Histamin ist und man unter inerter Atmosphäre und im Dunkeln arbeitet.

12. Verfahren gemäß einem der Ansprüche 8 bis 11, dadurch gekennzeichnet, daß das Ausgangsprodukt das 17β-Hydroxy-4,9,11-trien-3-on ist und man das Produkt der Formel

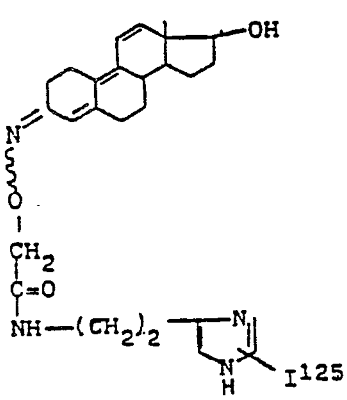

in Form eines Gemisches der syn- und anti-Isomeren oder in Form des syn-Isomeren oder in Form von anti-Isomeren erhält.

13. Verfahren gemäß einem der Ansprüche 8 bis 11, dadurch gekennzeichnet, daß das Ausgangsprodukt das 17α-Hydroxy-4,9,11-trien-3-on ist und man das Produkt der Formel

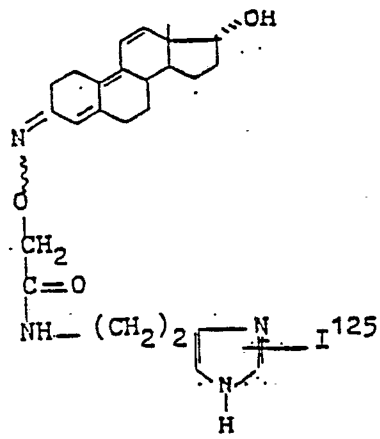

in Form eines Gemisches der syn- und anti-Isomeren oder in Form des syn-Isomeren oder in Form des anti-Isomeren erhält.

14. Verwendung der Produkte gemäß einem der Ansprüche 1 bis 7 für radio-immunologische Bestimmungen der 17β- und 17α-Hydroxy-4,9,11-trien-3-one in biologischen Flüssigkeiten und Geweben bei der menschlichen und tierischen Ernährung.

15. Als Zwischenprodukte, die für die Herstellung der Produkte der allgemeinen Formel I gemäß den Ansprüchen 1 bis 7 erforderlich sind, die Produkte der allgemeinen Formel III in Form eines Gemisches der syn- und anti-Isomeren oder in Form des syn-Isomeren oder in Form des anti-Isomeren

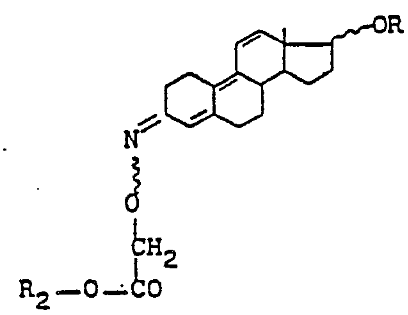

(III)

worin die gewellten Linien anzeigen, daß sich die OR-Funktion in α- oder β-Stellung befinden kann und sich die Oximfunktion in syn- oder anti-Stellung befinden kann, und worin R ein Wasserstoffatom, eine Alkylgruppe mit 1 bis 6 Kohlenstoffatomen oder eine Acylgruppe einer Carboxylsäure mit 2 bis 12 Kohlenstoffatomen bedeutet, $R_2$ ein Wasserstoffatom, eine Alkylgruppe mit 1 bis 6 Kohlenstoffatomen oder eine Aktivatorgruppe für die Carbonylfunction der Formel —CO—C—alk, worin alk einen Alkylrest mit 1 bis 6 Kohlenstoffatomen darstellt, bedeutet.

16. Die Produkte gemäß Anspruch 15 mit den folgenden Bezeichnungen :

— 17β-Hydroxy-4,9,11-trien-3-carboxymethyloxim in Form eines Gemisches der syn- und anti-Isomeren, in Form des syn-Isomeren und in Form des anti-Isomeren ;

— 17α-Hydroxy-4,9,11-trien-3-carboxymethyloxim in Form eines Gemisches der syn- und anti-Isomeren, in Form des syn-Isomeren und in Form des anti-Isomeren ;

— 17β-Hydroxy-4,9,11-trien-3-carbethoxymethyloxim in Form eines Gemisches der syn- und anti-Isomeren, in Form des syn-Isomeren und in Form des anti-Isomeren ;

— 17α-Hydroxy-4,9,11-trien-3-carbethoxymethyloxim in Form eines Gemisches der syn- und anti-Isomeren, in Form des syn-Isomeren und in Form des anti-Isomeren ;

— gemischtes Anhydrid von 17β-Hydroxy-4,9,11-trien-3-carboxymethyloxim mit dem Isobutylformiat in Form eines Gemisches des syn- und anti-Isomeren, in Form des syn-Isomeren und in Form des anti-Isomeren :

(Siehe Formel Seite 32 f.)

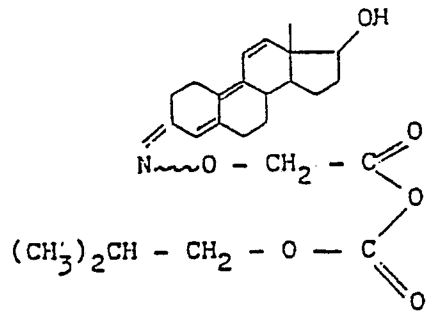

worin die gewellte Linie anzeigt, daß sich die Oximfunktion in der Stellung des syn-Isomeren oder in der Stellung des anti-Isomeren befinden kann oder ein Gemisch dieser beiden Isomeren darstellen kann ;

— das gemischte Anhydrid von 17$\alpha$-Hydroxy-4,9,11-trien-3-carboxymethyloxim mit Isobutylformiat in Form eines Gemisches des syn- und anti-Isomeren in Form des syn-Isomeren und in Form des anti-Isomeren

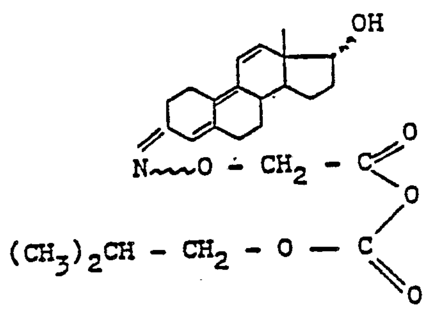

worin die gewellte Linie anzeigt, daß sich die Oximfunktion in der Stellung des syn-Isomerne oder in der Stellung des anti-Isomeren befinden kann oder ein Gemisch dieser beiden Isomeren darstellen kann.

17. Antigene, bestehend aus der Konjugation eines Produkts der allgemeinen Formel III gemäß Anspruch 15, worin $R_2$ ein Wasserstoffatom bedeutet, mit Rinderserumalbumin (BSA) oder mit Humanserumalbumin (HSA) der Formeln

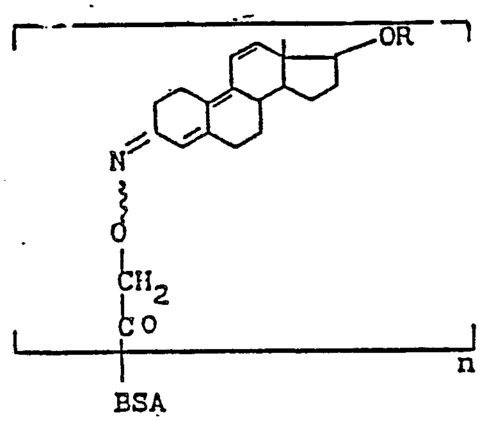

(IV)

worin n = 20 bis 30 und worin der Oximteil des Steroids in Form des syn-Isomeren, des anti-Isomeren oder in Form eines Gemisches der syn- und anti-Isomeren vorliegt ;

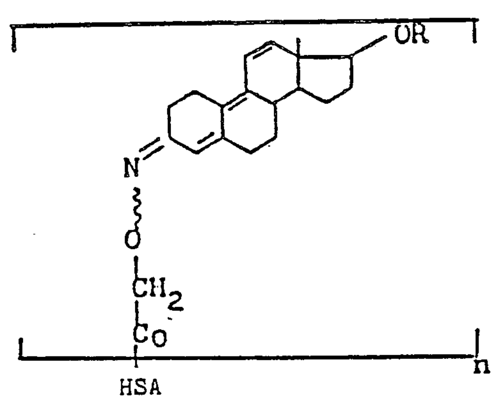

(V)

worin n = 20 bis 30 und worin der Oximteil des Steroids in Form des syn-Isomeren, in Form des anti-Isomeren oder in Form eines Gemisches der syn- und anti-Isomeren vorliegt;

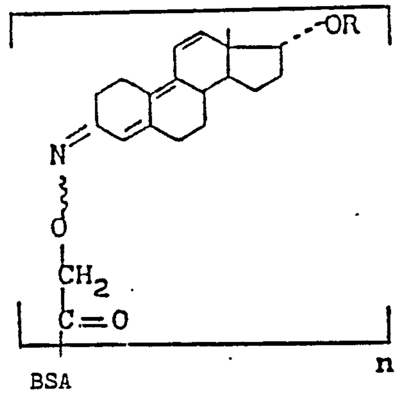

(VI)

worin n = 20 bis 30 und worin der Oximteil des Steroids in Form des syn-Isomeren, in Form des anti-Isomeren oder in Form eines Gemisches der syn- und anti-Isomeren vorliegt;

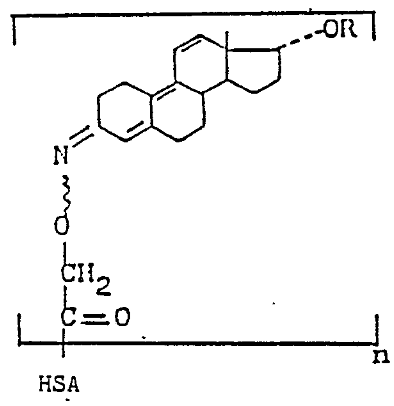

(VII)

worin n = 20 bis 30 und worin der Oximteil des Steroids in Form des syn-Isomeren, in Form des anti-Isomeren und in Form des Gemisches der syn- und anti-Isomeren vorliegt.

18. Verfahren zur Herstellung der Antigene gemäß Anspruch 17, dadurch gekennzeichnet, daß man ein Produkt der allgemeinen Formel III, wie in Anspruch 15 definiert, worin $R_2$ ein Wasserstoffatom bedeutet, mit Rinderserumalbumin (BSA) oder mit Humanserumalbumin (HSA) konjugiert und das gemischte Antigen erhält.

19. Verfahren gemäß Anspruch 18, dadurch gekennzeichnet, daß man ein Produkt der allgemeinen Formel $III_A$ in Form eines Gemisches des syn- und anti-Isomeren, in Form des syn-Isomeren oder in Form des anti-Isomeren.

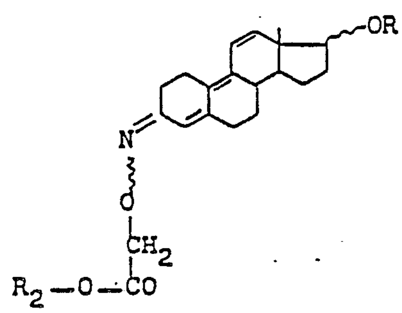

($III_A$)

worin die gewellten Linien und R die vorstehenden Bedeutungen besitzen und $R_2$ ein Wasserstoffatom darstellt, im Hinblick auf die Aktivierung der Carbonylfunction mit einem Alkylhalogenoformiat in Anwesenheit einer tertiären Base in wasserfreiem Milieu und unter inerter Atmosphäre umsetzt, das entsprechende, gemischte Anhydrid der Formel $III_C$

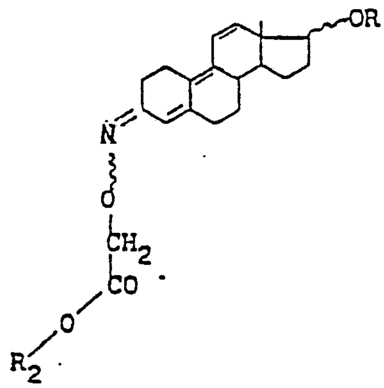

(IIIc)

worin $R_2$ einen Rest der Formel —CO—O—alc bedeutet, worin alc einen Alkylrest mit höchstens 6 Kohlenstoffatomen darstellt, erhält, welches man mit Rinderserumalbumin (BSA) oder Humanserumalbumin (HSA) konjugiert und das erwünschte Antigen erhält.

20. Verfahren gemäß Anspruch 19, dadurch gekennzeichnet, daß

— das Alkylhalogenoformiat Isobutylchloroformiat ist und man in Anwesenheit von Tri-n-butylamin und unter inerter Atmosphäre arbeitet,

— man das gemischte Anhydrid der allgemeinen Formel $III_C$ mit Rinderserumalbumin (BSA) oder Humanserumalbumin (HSA) umsetzt, indem man zuvor dieses letztere in einem Wasser-Dioxan-Gemisch unter inerter Atmosphäre gelöst hat.

21. Verwendung der Antigene gemäß 17 zur Herstellung von Antikörpern.